# EUROPEAN PATENT APPLICATION

(11) **EP 3 663 311 A1**
(43) Date of publication of application: **10.06.2020**
(21) Application number: 18382882.1
(22) Date of filing: 03.12.2018
(51) Int. Cl.: C07K 14/245, C07K 14/415, G01N 33/68

(54) **CHIMERIC PROTEIN SWITCH FOR THE OPTOGENETIC CONTROL OF AMYLOIDOGENESIS**

(71) Applicant: Consejo Superior de Investigaciones Científicas (CSIC), 28006 Madrid (ES)
(72) Inventor: GIRALDO SUÁREZ, Rafael, 28040 Madrid (ES)
(74) Representative: Pons Ariño, Angel

(57) **Abstract**

The present invention provides an optogenetic chimeric fusion polypeptide comprising an optimized amino acid sequence of the plant phototropin domain LOV2 fused to an amino acid sequence of the bacterial amyloidogenic effector RepA-WH1. Optimized LOV2 enables navigation through the folding landscape of RepA-WH1 from solubility to its aggregation as oligomers or amyloid fibres. Thus, this polypeptide assembles as hydrogels and amyloid fibres in the darkness, while under blue light illumination forms oligomeric particles that are proteotoxic for cells, preferably bacteria. This polypeptide is therefore proposed for inducing the formation of cytotoxic amyloid oligomers in cells that are targeted for killing.

SEQUENCE LISTING

<110>  Consejo Superior de Investigaciones Científicas (CSIC)

<120>  CHIMERIC PROTEIN SWITCH FOR THE OPTOGENETIC CONTROL OF
       AMYLOIDOGENESIS

<130>  EP1641.1408

<160>  11

<170>  PatentIn version 3.5

<210>  1
<211>  285
<212>  PRT
<213>  Artificial Sequence

<220>
<223>  LOV543m3-WH1 chimera

<400>  1

Met Gly Ser Ser His His His His His His His His His His Ser Ser
1               5                   10                  15

Gly Leu Val Pro Arg Gly Ser Leu Ala Thr Thr Leu Glu Arg Ile Glu
            20                  25                  30

Lys Asn Phe Val Ile Thr Asp Pro Arg Leu Pro Asp Asn Pro Ile Ile
        35                  40                  45

Phe Ala Ser Asp Ser Phe Leu Gln Leu Thr Glu Tyr Ser Arg Glu Glu
    50                  55                  60

Ile Leu Gly Arg Asn Cys Arg Phe Leu Gln Gly Pro Glu Thr Asp Arg
65                  70                  75                  80

Ala Thr Val Arg Lys Ile Arg Asp Ala Ile Asp Asn Gln Thr Glu Val
                85                  90                  95

Thr Val Gln Leu Ile Asn Tyr Thr Lys Ser Gly Lys Lys Phe Trp Asn
            100                 105                 110

Leu Phe His Leu Gln Pro Met Arg Asp Gln Lys Gly Asp Val Gln Tyr
        115                 120                 125

Phe Ile Gly Val Gln Leu Asp Gly Thr Glu His Val Arg Asp Ala Ala
    130                 135                 140

Glu Arg Glu Ala Val Met Glu Ala Lys Lys Thr Ala Glu Asn Ile Asp
145                 150                 155                 160

Glu Ala Ala Lys Leu Ile Glu Ser Ser His Thr Leu Thr Leu Asn Glu
                165                 170                 175

Lys Arg Leu Val Leu Cys Ala Ala Ser Leu Ile Asp Ser Arg Lys Pro
            180                 185                 190

Leu Pro Lys Asp Gly Tyr Leu Thr Ile Arg Ala Asp Thr Phe Ala Glu
        195                 200                 205

Val Phe Gly Ile Asp Val Lys His Ala Tyr Ala Ala Leu Asp Asp Ala
    210                 215                 220

Ala Thr Lys Leu Phe Asn Arg Asp Ile Arg Arg Tyr Val Lys Gly Lys
225                 230                 235                 240

Val Val Glu Arg Met Arg Trp Val Phe His Val Lys Tyr Arg Glu Gly
                245                 250                 255

Gln Gly Cys Val Glu Leu Gly Phe Ser Pro Thr Ile Ile Pro His Leu
            260                 265                 270

Thr Met Leu His Lys Glu Phe Thr Ser Tyr Gln Leu Lys
        275                 280                 285

<210>  2
<211>  236
<212>  PRT
<213>  Artificial Sequence

<220>
<223>  mCherry fluorescent protein probe

<400>  2

Met Val Ser Lys Gly Glu Glu Asp Asn Met Ala Ile Ile Lys Glu Phe
1               5                   10                  15

Met Arg Phe Lys Val His Met Glu Gly Ser Val Asn Gly His Glu Phe
            20                  25                  30

Glu Ile Glu Gly Glu Gly Glu Gly Arg Pro Tyr Glu Gly Thr Gln Thr
        35                  40                  45

Ala Lys Leu Lys Val Thr Lys Gly Gly Pro Leu Pro Phe Ala Trp Asp
    50                  55                  60

Ile Leu Ser Pro Gln Phe Met Tyr Gly Ser Lys Ala Tyr Val Lys His
65                  70                  75                  80

Pro Ala Asp Ile Pro Asp Tyr Leu Lys Leu Ser Phe Pro Glu Gly Phe

85                  90                  95

Lys Trp Glu Arg Val Met Asn Phe Glu Asp Gly Gly Val Val Thr Val
            100                 105                 110

Thr Gln Asp Ser Ser Leu Gln Asp Gly Glu Phe Ile Tyr Lys Val Lys
        115                 120                 125

Leu Arg Gly Thr Asn Phe Pro Ser Asp Gly Pro Val Met Gln Lys Lys
    130                 135                 140

Thr Met Gly Trp Glu Ala Ser Ser Glu Arg Met Tyr Pro Glu Asp Gly
145                 150                 155                 160

Ala Leu Lys Gly Glu Ile Lys Gln Arg Leu Lys Leu Lys Asp Gly Gly
                165                 170                 175

His Tyr Asp Ala Glu Val Lys Thr Thr Tyr Lys Ala Lys Lys Pro Val
            180                 185                 190

Gln Leu Pro Gly Ala Tyr Asn Val Asn Ile Lys Leu Asp Ile Thr Ser
        195                 200                 205

His Asn Glu Asp Tyr Thr Ile Val Glu Gln Tyr Glu Arg Ala Glu Gly
    210                 215                 220

Arg His Ser Thr Gly Gly Met Asp Glu Leu Tyr Lys
225                 230                 235

<210>  3
<211>  528
<212>  PRT
<213>  Artificial Sequence

<220>
<223>  LOV543m3-WH1-mCherry chimera

<400>  3

Met Gly Ser Ser His His His His His His His His His His Ser Ser
1               5                   10                  15

Gly Leu Val Pro Arg Gly Ser Leu Ala Thr Thr Leu Glu Arg Ile Glu
            20                  25                  30

Lys Asn Phe Val Ile Thr Asp Pro Arg Leu Pro Asp Asn Pro Ile Ile
        35                  40                  45

Phe Ala Ser Asp Ser Phe Leu Gln Leu Thr Glu Tyr Ser Arg Glu Glu
    50                  55                  60

Ile Leu Gly Arg Asn Cys Arg Phe Leu Gln Gly Pro Glu Thr Asp Arg
65                  70                  75                  80

Ala Thr Val Arg Lys Ile Arg Asp Ala Ile Asp Asn Gln Thr Glu Val
                85                  90                  95

Thr Val Gln Leu Ile Asn Tyr Thr Lys Ser Gly Lys Lys Phe Trp Asn
            100                 105                 110

Leu Phe His Leu Gln Pro Met Arg Asp Gln Lys Gly Asp Val Gln Tyr
        115                 120                 125

Phe Ile Gly Val Gln Leu Asp Gly Thr Glu His Val Arg Asp Ala Ala
    130                 135                 140

Glu Arg Glu Ala Val Met Glu Ala Lys Lys Thr Ala Glu Asn Ile Asp
145                 150                 155                 160

Glu Ala Ala Lys Leu Ile Glu Ser Ser His Thr Leu Thr Leu Asn Glu
                165                 170                 175

Lys Arg Leu Val Leu Cys Ala Ala Ser Leu Ile Asp Ser Arg Lys Pro
            180                 185                 190

Leu Pro Lys Asp Gly Tyr Leu Thr Ile Arg Ala Asp Thr Phe Ala Glu
        195                 200                 205

Val Phe Gly Ile Asp Val Lys His Ala Tyr Ala Ala Leu Asp Asp Ala
    210                 215                 220

Ala Thr Lys Leu Phe Asn Arg Asp Ile Arg Arg Tyr Val Lys Gly Lys
225                 230                 235                 240

Val Val Glu Arg Met Arg Trp Val Phe His Val Lys Tyr Arg Glu Gly
                245                 250                 255

Gln Gly Cys Val Glu Leu Gly Phe Ser Pro Thr Ile Ile Pro His Leu
            260                 265                 270

Thr Met Leu His Lys Glu Phe Thr Ser Tyr Gln Leu Lys Gly Ser Ser
        275                 280                 285

Gly Ser Ser Gly Met Val Ser Lys Gly Glu Glu Asp Asn Met Ala Ile
    290                 295                 300

Ile Lys Glu Phe Met Arg Phe Lys Val His Met Glu Gly Ser Val Asn

305                 310                 315                 320

Gly His Glu Phe Glu Ile Glu Gly Glu Gly Glu Gly Arg Pro Tyr Glu
                325                 330                 335

Gly Thr Gln Thr Ala Lys Leu Lys Val Thr Lys Gly Gly Pro Leu Pro
            340                 345                 350

Phe Ala Trp Asp Ile Leu Ser Pro Gln Phe Met Tyr Gly Ser Lys Ala
        355                 360                 365

Tyr Val Lys His Pro Ala Asp Ile Pro Asp Tyr Leu Lys Leu Ser Phe
    370                 375                 380

Pro Glu Gly Phe Lys Trp Glu Arg Val Met Asn Phe Glu Asp Gly Gly
385                 390                 395                 400

Val Val Thr Val Thr Gln Asp Ser Ser Leu Gln Asp Gly Glu Phe Ile
                405                 410                 415

Tyr Lys Val Lys Leu Arg Gly Thr Asn Phe Pro Ser Asp Gly Pro Val
            420                 425                 430

Met Gln Lys Lys Thr Met Gly Trp Glu Ala Ser Ser Glu Arg Met Tyr
        435                 440                 445

Pro Glu Asp Gly Ala Leu Lys Gly Glu Ile Lys Gln Arg Leu Lys Leu
    450                 455                 460

Lys Asp Gly Gly His Tyr Asp Ala Glu Val Lys Thr Thr Tyr Lys Ala
465                 470                 475                 480

Lys Lys Pro Val Gln Leu Pro Gly Ala Tyr Asn Val Asn Ile Lys Leu
                485                 490                 495

Asp Ile Thr Ser His Asn Glu Asp Tyr Thr Ile Val Glu Gln Tyr Glu
            500                 505                 510

Arg Ala Glu Gly Arg His Ser Thr Gly Gly Met Asp Glu Leu Tyr Lys
        515                 520                 525

<210>  4
<211>  140
<212>  PRT
<213>  Artificial Sequence

<220>
<223>  LOV543m3 chimera

<400>  4

Leu Ala Thr Thr Leu Glu Arg Ile Glu Lys Asn Phe Val Ile Thr Asp
1               5                   10                  15

Pro Arg Leu Pro Asp Asn Pro Ile Ile Phe Ala Ser Asp Ser Phe Leu
            20                  25                  30

Gln Leu Thr Glu Tyr Ser Arg Glu Glu Ile Leu Gly Arg Asn Cys Arg
        35                  40                  45

Phe Leu Gln Gly Pro Glu Thr Asp Arg Ala Thr Val Arg Lys Ile Arg
    50                  55                  60

Asp Ala Ile Asp Asn Gln Thr Glu Val Thr Val Gln Leu Ile Asn Tyr
65                  70                  75                  80

Thr Lys Ser Gly Lys Lys Phe Trp Asn Leu Phe His Leu Gln Pro Met
                85                  90                  95

Arg Asp Gln Lys Gly Asp Val Gln Tyr Phe Ile Gly Val Gln Leu Asp
            100                 105                 110

Gly Thr Glu His Val Arg Asp Ala Ala Glu Arg Glu Ala Val Met Glu
        115                 120                 125

Ala Lys Lys Thr Ala Glu Asn Ile Asp Glu Ala Ala
    130                 135                 140


<210>  5
<211>  122
<212>  PRT
<213>  Artificial Sequence

<220>
<223>  RepA-WH1 protein

<400>  5

Lys Leu Ile Glu Ser Ser His Thr Leu Thr Leu Asn Glu Lys Arg Leu
1               5                   10                  15

Val Leu Cys Ala Ala Ser Leu Ile Asp Ser Arg Lys Pro Leu Pro Lys
            20                  25                  30

Asp Gly Tyr Leu Thr Ile Arg Ala Asp Thr Phe Ala Glu Val Phe Gly
        35                  40                  45

Ile Asp Val Lys His Ala Tyr Ala Ala Leu Asp Asp Ala Ala Thr Lys
    50                  55                  60

Leu Phe Asn Arg Asp Ile Arg Arg Tyr Val Lys Gly Lys Val Val Glu
65                  70                  75                  80

Arg Met Arg Trp Val Phe His Val Lys Tyr Arg Glu Gly Gln Gly Cys
                85                  90                  95

Val Glu Leu Gly Phe Ser Pro Thr Ile Ile Pro His Leu Thr Met Leu
            100                 105                 110

His Lys Glu Phe Thr Ser Tyr Gln Leu Lys
        115                 120

<210>  6
<211>  420
<212>  DNA
<213>  Avena sativa

<400>  6
cttgctacta cacttgaacg tattgagaag aactttgtca ttactgaccc acgtttgcca      60

gataatccca ttatcttcgc gtccgatagt ttcttgcagt tgacagaata ttcgcgagaa     120

gaaattctgg gtcgtaactg ccgttttctt caaggtcctg aaaccgatcg cgcgacagtg     180

cgcaaaattc gtgatgccat cgataaccaa acagaggtca ctgtacagct gattaattat     240

acaaagagtg gtaaaaagtt ctggaacctc tttcacttgc agcctatgcg tgatcagaag     300

ggtgatgtcc agtactttat tggtgtccag ttggatggta ccgaacatgt ccgtgatgcg     360

gccgagcgtg agggtgtcat gctgattaag aaaactgcag aaaatattga tgaggcggca     420

<210>  7
<211>  426
<212>  DNA
<213>  Artificial Sequence

<220>
<223>  Codon optimized sequence from SEQ ID NO: 6

<400>  7
atgctggcga ccaccctgga acgtattgaa aaaaactttg tgattaccga tccgcgtctg      60

ccggataacc cgattatttt tgcgagcgat agctttctgc agctgaccga atatagccgt     120

gaagaaattc tgggccgtaa ctgccgtttt ctgcagggcc cggaaaccga tcgtgcgacc     180

gtgcgtaaaa ttcgtgatgc gattgataac cagaccgaag tgaccgtgca gctgattaac     240

tataccaaaa gcggcaaaaa attttggaac ctgtttcatc tgcagccgat gcgtgatcag     300

aaaggtgatg tccagtactt tattggtgtc cagttggatg gtaccgaaca tgtccgtgat     360

gcggcggaac gtgaaggcgt gatgctgatt aaaaaaaccg cagaaaacat tgatgaagcg     420

gcgtaa                                                                426

<210>  8
<211>  59
<212>  DNA
<213>  Artificial Sequence

<220>
<223>  Primer G528A_I532A-F

<400>  8
gtgatgcggc ggaacgtgaa gccgtgatgc tggctaaaaa aaccgcagaa aacattgat          59


<210>  9
<211>  59
<212>  DNA
<213>  Artificial Sequence

<220>
<223>  Primer G528A_I532A-R

<400>  9
atcaatgttt tctgcggttt ttttagccag catcacggct tcacgttccg ccgcatcac          59


<210>  10
<211>  45
<212>  DNA
<213>  Artificial Sequence

<220>
<223>  Primer G528A_L531E_I532A-F

<400>  10
cggaacgtga agccgtgatg gaggctaaaa aaaccgcaga aaaca                         45


<210>  11
<211>  45
<212>  DNA
<213>  Artificial Sequence

<220>
<223>  Primer G528A_L531E_I532A-R

<400>  11
tgttttctgc ggttttttta gcctccatca cggcttcacg ttccg                         45

## Description

This invention belongs to the fields of amyloidosis, optogenetics and protein synthetic conformational switches. In particular, this invention refers to a chimeric fusion protein based on the photoreceptor domain LOV2 fused to an effector protein with amyloidogenic potential. This chimeric protein allows the light-control of amyloidogenesis within cells, since the excitation of the protein with blue light leads to the formation of cytotoxic soluble amyloid aggregates or oligomers in the cell expressing the chimeric protein. This kind of light-switchable fusion proteins have multiple applications, for example, as antimicrobial agents based on triggering amyloidosis within undesired bacterial cells.

### BACKGROUND ART

Through the absorption of photons by chromogenic prosthetic groups, light has the capacity to elicit conformational re-arrangements in natural effector proteins. An emerging discipline, Optogenetics, has implemented the engineering of such photoreceptors to create synthetic conformational switches that much expand the functional abilities of proteins (Khamo, J. S., et al., 2017, J. Mol. Biol., 429, 2999-3017). One of the most used photoreceptors in optogenetics is the LOV domain, with many variants widespread across the whole phylogenetic tree (Glantz, S. T., et al., 2016, Proc. Natl. Acad. Sci. USA, 113, E1442-E1451). One of the LOV domains most commonly used in optogenetic is the plant photoreceptor LOV2 (Zimmerman, S. P., et al., 2016, Methods Enzymol., 580, 169-190).

Engineering chimeric proteins to become light-responsive switches has recently enabled physical control on cellular processes such as channel gating, gene expression, organelle targeting and proteolysis. The design of protein chimeras including a light-responsive domain, which couples conformational changes, with a functional switch in its fusion partner, has equipped Synthetic Biology with powerful optogenetic tools to get physical control on a plethora of cellular processes. Thus, over more than a decade, optogenetic parts have been designed, and successfully assayed, to govern ion fluxes across membrane channels and thus neuronal circuits, cytoskeleton dynamics and nuclear localization, membrane trafficking, allosteric photo-regulation of enzyme catalysis, apoptosis through activating caspases, protein degradation by ubiquitinilation, or gene expression through regulation of the binding of proteins to DNA. In many of these instances, effector peptide tags became exposed upon illumination of cells with light of a wavelength (UV/Vis/IR) matching an absorption band in a chromophoric prosthetic group bound to the light-responsive domain.

LOV-based optogenetic tools have been gaining wide popularity in recent years to control a myriad of cellular events, including cell motility (Wu, Yi I., et al., 2009, Nature, 461 (7260): 104-8), subcellular organelle distribution (van Bergeijk, et al., 2015, Nature, 518 (7537): 111-4), formation of membrane contact sites (Jing, Ji, et al., 2015, Nature Cell Biology, 17(10): 1339-47), and protein degradation (Renicke, Christian, et al., 2013, Chemistry & Biology, 20 (4): 619-626).

However, no optogenetic protein tool has been described having direct control on the aggregation pathways leading to the assembly of amyloids. Thus, amyloid opto(epi)genetics remains yet unexplored.

Amyloids, which are among the most stable natural macromolecular structures, are made of β-strand segments from different individual molecules of a given protein that assemble, through intermediate oligomeric states, into mature fibres. Amyloidogenesis is readily accessible to short peptides or intrinsically disordered protein domains but, for fully folded proteins with a stable three-dimensional fold, partial unfolding to a metastable state is mandatory, which is usually attained through disease-linked destabilizing mutations *in vivo* or by resorting to harsh physical-chemical conditions *in vitro.*

RepA-WH1 (Giraldo, R., et al., 2016, Prion, 10, 41-49) is a manifold domain from a plasmid-encoded bacterial protein that undergoes conformational changes that capacitate it either as a transcriptional repressor, as a DNA replication initiator or, through its assembly as amyloid oligomers, to hinder premature replication rounds. Although very stable in solution, RepA-WH1 dimers become metastable upon binding to dsDNA or acidic phospholipids, thus paving the pathway towards amyloidogenesis. In the bacterial cytoplasm, the fusion of a hyper-amyloidogenic mutant variant of RepA-WH1 to the fluorescent protein mCherry generates a prion-like protein (prionoid) that epigenetically propagates from mother-to-daughter cells, causing a synthetic amyloid proteinopathy (Fernández-Tresguerres, M. E., et al., 2010, Microbiol., 77, 1456-1469). Thus, RepA-WH1 was previously engineered to boost amyloidogenicity and uncouple its conformational remodelling from its natural function, thus generating the only intracellular proteinopathy described so far in bacteria, which has been useful as a minimal model to deconstruct a 'generic' amyloid disease (Fernández, C., et al., 2016, Sci. Rep., 6, 23144).

RepA-WH1 amyloidosis recapitulates some of the hallmarks of the mitochondrial damage associated with human amyloid diseases, including the formation oligomeric pores at the internal membrane, the generation of reactive oxygen species (ROS) and the loss of function, due to co-aggregation, of essential cell factors. In addition, RepA-WH1 has been used as a bench proof for the design of synthetic tools to probe protein amyloidogenesis, including gold nanoparticle-based sensors (Fernández, C., et al., 2016, Angew. Chem. Int. Ed., 55, 11237-11241) and screening devices based on the disruption of protein translation either in yeast (Gasset-Rosa, F. & Giraldo, R., 2015, Front. Microbiol., 6, 311) or in bacteria (Molina-Garcia, L. & Giraldo, R., 2017, Sci. Rep., 7, 11908).

However, there is a need for optogenetic protein tools that allow the direct control on the assembly of cytotoxic amyloid fibres or oligomers which are useful for triggering amyloidosis within undesired cells that need to be killed.

### DESCRIPTION OF THE INVENTION

The present invention provides an optogenetic chimeric fusion polypeptide comprising an optimized (mutated) amino acid sequence of the plant phototropin LOV2 domain fused to an amino acid sequence of the bacterial amyloidogenic effector RepA-WH1. Optimized LOV2 enables navigation through the folding landscape of RepA-WH1 from solubility to its aggregation as oligomers or amyloid fibres. Thus, this designed polypeptide assembles as hydrogels and amyloid fibres in the darkness, while under blue light illumination forms oligomeric particles that are proteotoxic for cells, preferably bacteria.

Here it is shown the feasibility of using optogenetic protein switches to surf the conformational landscape of a protein on a pathway leading to amyloidosis. In this invention optogenetic control on a phase transition leading to the assembly of amyloid fibres or oligomers with characteristic toxicity has been achieved.

This invention describes therefore the construction of a blue light-responsive chimera between an optimized plant phototropin LOV2 domain and the bacterial prion-like protein RepA-WH1. In the darkness, and in a crowded environment *in vitro,* this chimera exhibits low sensitivity to proteases and is competent to nucleate on RepA-WH1 the assembly of amyloid fibres and hydrogels. When expressed in *Escherichia coli,* this chimera forms in the darkness large intracellular amyloid aggregates. However, under blue light illumination the same chimera has increased sensitivity to proteolysis and templates the assembly of discrete oligomeric RepA-WH1 particles and liquid droplets *in vitro.* Such lit-state oligomers exhibit enhanced cytotoxicity *in vivo.*

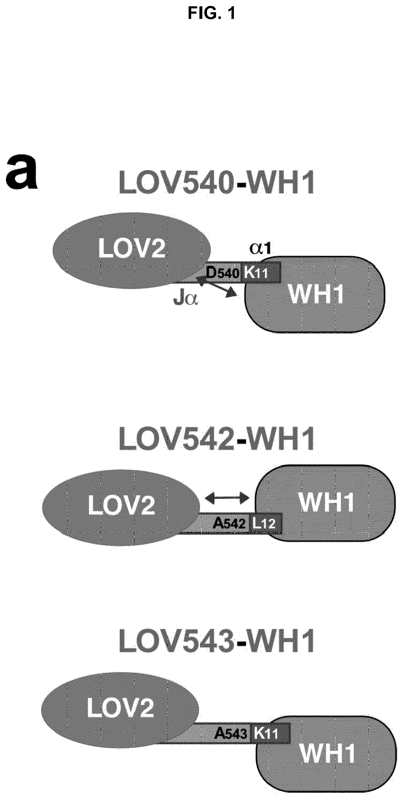

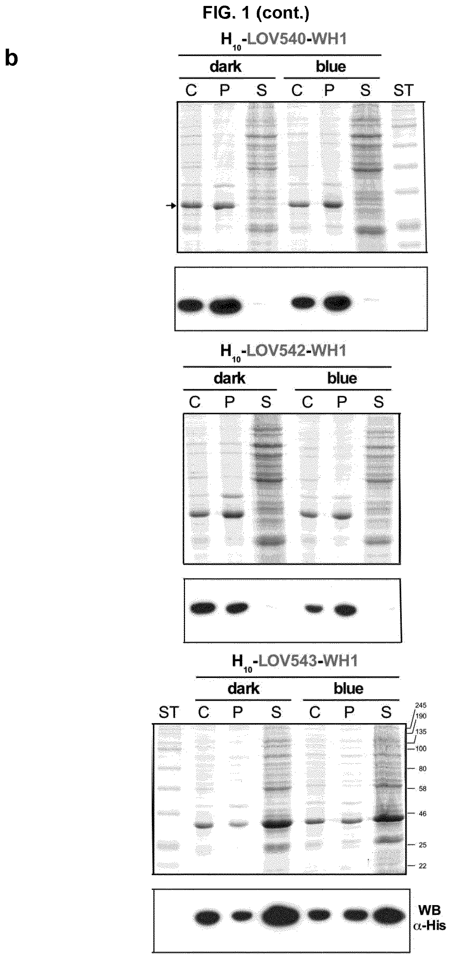

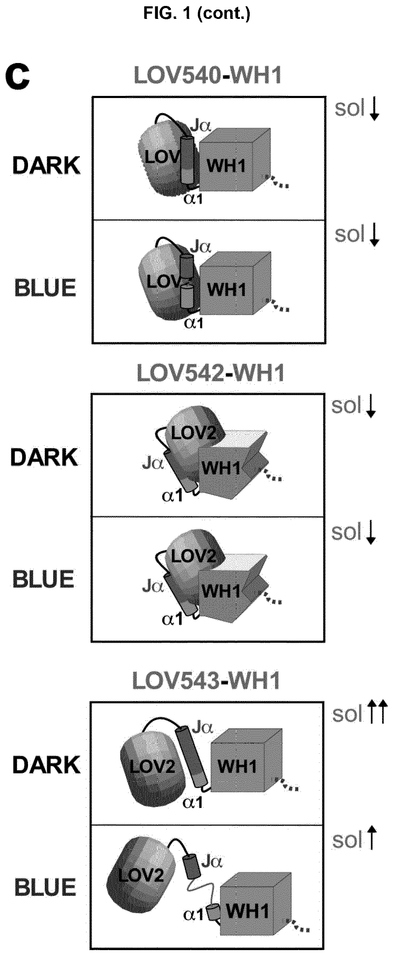

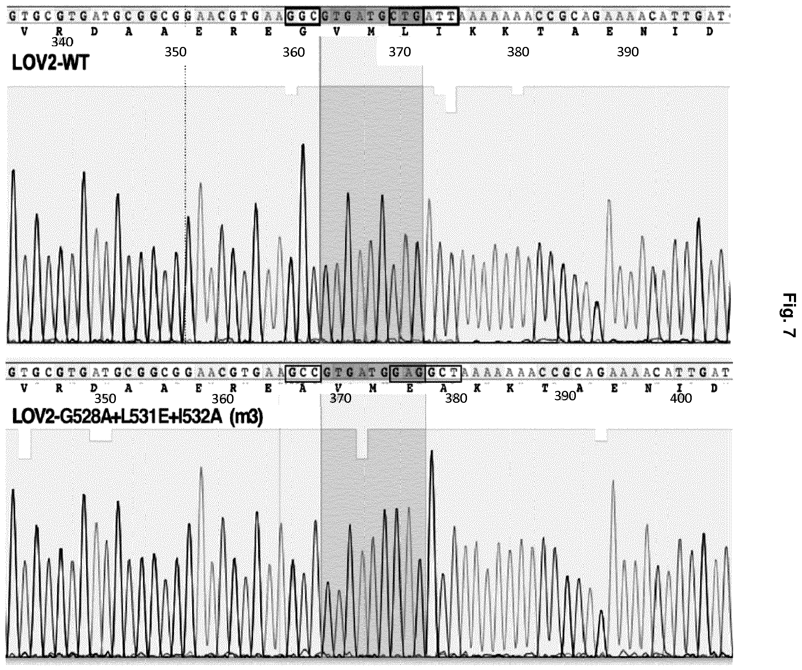

The chimeric protein described in this invention was first optimized by modulating the phase and length of the linker Jα-α1 helix (LOV₅₄₃-WH1₁₁; see Fig. 1), plus the inclusion of mutations that stabilize the dark state conformation of Jα (Figs. 7-9).

The chimera presented in this invention enriches and expands the catalogue of available optogenetic tools with a novel way to guide the conformational landscape of proteins towards amyloidogenesis. Its applications include, without limitations:
- Controlling the assembly of amyloid nanoscaffolds to engage enzymes in sequential reaction steps. Nanoscaffolds are protein architectures (fibrilar, tubular, laminar, icosahedral, gel-like) to which other proteins, nucleic acids or functionalized organic molecules can bind to enhance their biological (e.g., catalytic) activities by the force of their immobilization, densities and intermolecular channelling.
- Building transcriptional switches for synthetic gene expression circuits and light-controlled plasmid replication cassettes. The conditional, light-dependent activation or inactivation of transcription factors would enable to block or trigger the synthesis of mRNA; whereas the effect of light on a DNA replication factor would do the same on the synthesis and propagation of a genetic mobile element (e.g., a plasmid). Achieving control on both, the expression/repression of a gene of interest and the propagation of the vector that carries it, are central biotechnological goals.
- The selective elimination of particular bacteria within a consortium, for instance once they have fulfilled their task in a bioprocess. In the design of bioprocesses, either for environmental or industrial applications, it is often crucial to get rid of bacteria after these have finished one bio-transformative step, in order to allow the proliferation and activity of a second different microorganism that might be outcompeted by the first. Light-enabled killing of the resident bacteria would provide an efficient way of achieving that goal.
- The development of a completely new kind of antimicrobials based on triggering amyloidosis by, e.g., bacteriophages encoding light-switchable cytotoxic amyloids. The emergence of antibiotic resistant bacteria currently is a major problem for human health worldwide. Thus the discovery of radically new ways to combat and kill pathogenic microorganisms is mandatory. Proteotoxic modules such as RepA-WH1, which could be delivered through bacteriophages (viruses), to kill bacteria upon light illumination would have a deep impact on clinics (e.g., in dermatological treatments against common skin pathogens such as *Staphylococcus aureus*).

Therefore, a first aspect of the present invention refers to a fusion polypeptide, hereinafter "the polypeptide of the invention", "the fusion protein of the invention", "the chimera of the invention" or "the chimeric protein of the invention", comprising the mutated amino acid sequence of the LOV2 domain shown in SEQ ID NO: 4 fused by its C-terminal end to the N-terminal end of the RepA-WH1 protein shown in SEQ ID NO: 5, wherein said fusion polypeptide comprises the amino acid sequence of SEQ ID NO: 1.

SEQ ID NO: 1 of the present invention is the chimera or mutant also called "LOV543m3-WH1" or "LOV2m3-WH1".

The "LOV2 domain" refers to the plant phototropin LOV2 domain or plant LOV2 photoreceptor. "LOV" means "Light-oxygen-voltage-sensing domain" and it is a protein sensor used by a large variety of higher plants, microalgae, fungi and bacteria to sense environmental conditions. In higher plants, it is used to control phototropism, chloroplast relocation, and stomatal opening. It has a blue-light sensitive flavin chromophore, which in the signaling state is covalently linked to the protein core via an adjacent cysteine residue. LOV domains are e.g. encountered in phototropins, which are blue-light-sensitive protein complexes regulating a great diversity of biological processes in higher plants as well as in microalgae. Phototropins are composed of two LOV domains, each containing a non-covalently bound flavin mononucleotide (FMN) chromophore in its dark-state form, and a C-terminal Ser-Thr kinase. Upon blue-light absorption, a covalent bond between the FMN chromophore and an adjacent reactive cysteine residue of the apo-protein is formed in the LOV2 domain. This subsequently mediates the activation of the kinase, which induces a signal in the organism through phototropin autophosphorylation.

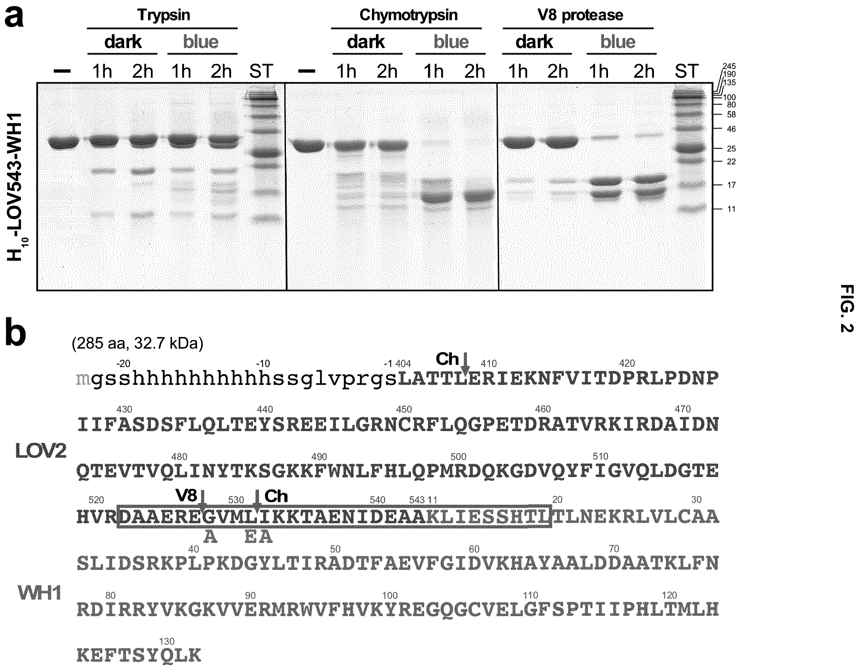

The "mutated amino acid sequence of the LOV2 domain" referred to in the present invention is the amino acid sequence shown in SEQ ID NO: 4 and it will be also called in the present invention "LOV543m3" or "LOV2m3". This SEQ ID NO: 4 comprises the amino acid substitutions G528A, L531E and I532A regarding the wild type amino acid sequence of the LOV2 domain (see Fig. 2). As shown in examples below, only this mutated variant of the LOV2 domain among those mutants tested by the inventor was light-responsive, i. e. differentially increased its solubility upon blue light illumination allowing thus the formation of cytotoxic amyloid oligomers.

In this invention, the term "LOV543wt" or "LOV2wt" refers to the LOV2 domain without the three amino acid substitutions indicated in the paragraph above. Thus, LOV543wt is the SEQ ID NO: 4 but comprising a G at position 528, a L at position 531 and an I at position 532 (see Figure 2).

The "RepA-WH1 protein" or "WH1" is the amino acid sequence shown in SEQ ID NO: 5.

The half-life of the lit (light-excited) state of the polypeptide of the invention is increased by 2.3-fold when the polypeptide is fused in its C-terminal end to the mCherry protein. Furthermore, this fusion to mCherry shows enhanced thermal stability in the polypeptide of the invention compared to the same polypeptide without mCherry. The fusion to mCherry also enhances in the polypeptide of the invention the metastability of RepA-WH1 and thus its amyloidogenicity.

Therefore, in a preferred embodiment, the amino acid sequence of SEQ ID NO: 1 is fused, in its C-terminal end, to the amino acid sequence of the mCherry fluorescent protein probe. More preferably, the amino acid sequence of the mCherry fluorescent protein probe is SEQ ID NO: 2.

In an even more preferably embodiment of this aspect of the invention, the polypeptide of the invention comprises the amino acid sequence SEQ ID NO: 3. In the most preferred embodiment of this aspect of the invention, the polypeptide of the invention consists of the amino acid sequence SEQ ID NO: 3.

SEQ ID NO: 3 of the present invention is the chimera of the invention also called "LOV543m3-WH1-mCherry" or "LOV2m3-WH1-mCherry".

The polypeptide of the invention may be produced by chemical synthesis or, as a recombinant, by an organism or cell that expresses a nucleotide sequence that encodes it. The polypeptide of the invention can be synthesised, for example, but without limitations, *in vitro.* For example, by means of the synthesis of solid-phase polypeptides or recombinant DNA approaches. It can be produced in a recombinant manner, including its production as a mature polypeptide or as a pre-protein that includes a signal peptide. Thus, the polypeptide of the invention may further comprise a signal peptide in its N-terminal end.

Another aspect of the invention refers to a nucleic acid sequence, hereinafter "the polynucleotide of the invention" or "the nucleic acid sequence of the invention", encoding the polypeptide of the invention.

Due to the degeneration of the genetic code, various nucleotide sequences can encode the same amino acid sequence.

In accordance with the present invention, a "nucleic acid molecule", "nucleotide sequence", "nucleic acid sequence" or "polynucleotide" is a nucleic acid molecule (polynucleotide) that has been extracted from its natural medium (i.e. it has been subjected to human manipulation) and can include DNA, RNA or DNA or RNA derivatives, including cDNA. The nucleotide sequence of the present invention may or may not be chemically or biochemically modified and can be artificially obtained by means of cloning and selection methods or by means of sequencing.

The polynucleotide sequence of the invention can encode the mature polypeptide or a pre-protein consisting of a signal peptide linked to the mature polypeptide that must be subsequently processed.

The polynucleotide sequence of the present invention may also comprise other elements, such as introns, non-encoding sequences at ends 3' and/or 5', ribosome binding sites, etc. This nucleotide sequence can also include encoding sequences for additional amino acids that are useful for the purification or stability of the encoded polypeptide.

The polynucleotide sequence of the invention can be included in a gene or genetic construct, preferably in a recombinant expression vector. Said genetic construct may also comprise one or more gene expression-regulating sequences, such as promoters, terminators, enhancers, etc.

Thus, another aspect of the invention refers to a genetic construct, hereinafter "the genetic construct of the invention", comprising the nucleic acid sequence of the invention, preferably wherein said genetic construct is an expression vector, more preferably a bacteriophage.

Alternatively, the expression vector referred to in the present invention may be a plasmid, preferably a low copy-number plasmid replicon, such as pRK2, pSC101, R1/F, or even multicopy plasmids (pUC, p15A, pBBR1), although others are not excluded.

The genetic construct of the invention may further comprise one or more sequences encoding for a specifically cleavable linker peptide functionally interposed between the mutated amino acid sequence of the LOV2 domain and the RepA-WH1 protein and/or between the RepA-WH1 protein and the mCherry protein. Such a linker peptide may be, for instance, a peptide sensitive to thrombin cleavage, factor X cleavage or other peptidase cleavage.

The genetic construct of the invention may further comprise one or more sequences encoding for purification tag/s linked to the polypeptide of the invention.

The genetic construct of the invention will generally be constructed such that the sequence encoding for the polypeptide of the invention is positioned adjacent to and under the control of an effective promoter. In certain cases, the promotor will comprise a prokaryotic promoter where the genetic construct is adapted for expression in a prokaryotic host cell. In other cases, the promoter will comprise a eukaryotic promoter where the genetic construct is adapted for expression in a eukaryotic host cell. In the later cases, when used for expression in eukaryotic hosts, the genetic construct will typically further include a polyadenylation signal at position 3' of the carboxy-terminal amino acid, and within a transcriptional unit of the encoded polypeptide. Promoters of particular utility in the genetic construct of the invention are bacterial promoters, preferably bacterial promoters functional in *E. coli* cells, such as for instance but without limitations, Ptac (IPTG/lactose-inducible), Ptet-ON/OFF (tetracyclin or doxicylin inducible/repressible), ParaBAD (arabinose-inducible) or PλCI^{ts} (temperature-inducible).

The expression "genetic construct", "gene construct" or "nucleic acid construct" as used herein relates to a functional unit required to transfer or express a nucleic acid sequence of interest, herein the nucleotide sequence of the invention as described, and regulatory sequences including, for example, a promoter, operably linked to the sequence that encodes the polypeptide, in an expression system. It refers to a nucleic acid molecule, mono or bicatenary, which is isolated from a natural gene or that is modified to contain nucleic acid segments in such a manner that they would otherwise not exist in nature. The expression "nucleic acid construct" is synonymous to the expression "expression cassette" when the construct of nucleic acid contains the control sequences required for the expression of the encoding sequence.

The term "expression vector", also known as "expression construct" or "plasmid", relates to a DNA molecule, linear or circular, that comprises the nucleic acid sequence of the invention operably linked to additional segments that provide the transcription of the encoded polypeptide. Generally, a plasmid is used to introduce a specific nucleic acid sequence in a target cell. Once the expression vector is in the interior of the cell, the protein encoded by the nucleic acid sequence is produced by means of the ribosome complexes of the cellular transcription and translation machinery. The plasmid is often subject to engineering to contain regulatory sequences that act as enhancer and promoter regions that lead to an efficient transcription of the nucleic acid sequence carried on the expression vector. The objective of a well-designed expression vector is the production of large amounts of stable messenger RNA and, therefore, of proteins. Expression vectors are basic tools for biotechnology and for the production of proteins, such as chimeric fusion proteins. The expression vector of the invention is introduced in a host cell such that the vector remains as a chromosome constituent or as an extra-chromosome self-replicating vector.

The term "expression" relates to the process whereby a polypeptide is synthesised from a polynucleotide. The term includes the transcription of the polynucleotide in a messenger RNA (mRNA) and the translation of said mRNA into a protein or polypeptide.

Examples of useful expression vectors are phages, cosmids, phagemids, yeast artificial chromosomes (YAC), bacterial artificial chromosomes (BAC), human artificial chromosomes (HAC) or viral vectors, such as adenovirus, baculovirus, retrovirus or lentivirus.

The polypeptide of the invention can be prepared using any known means in the state of the art, such as the transformation of the nucleic acid sequence of the invention in an adequate host cell and the expression of said sequence to obtain the polypeptide of the invention.

Another aspect of the invention refers to a cell, hereinafter "the cell of the invention", comprising the fusion polypeptide, the nucleic acid sequence or the genetic construct of the invention.

The cell of the invention may be either a eukaryotic or a prokaryotic cell. This cell is the recipient of an expression vector, cloning vector or any other DNA molecule. Therefore, it includes any cultivable cell that may be modified through the introduction of DNA not contained naturally therein. Preferably, this cell is that in which the polynucleotide of the invention may be expressed, giving rise to a stable polypeptide, post-translationally modified and located in the appropriate subcellular compartment. The election of an appropriate cell may also be influenced by the election of the detection signal. For example, the use of constructs with reporter genes (for example, *lacZ,* luciferase, thymidine kinase, green fluorescent protein "GFP" or red fluorescent protein "mCherry") can provide a signal selectable through the activation or inhibition of the transcription of the nucleotide sequence of interest in response to a transcription-regulating protein. In order to achieve optimum selection or screening, the phenotype of the cell must be considered.

In any case, the polynucleotide or the genetic construct of the invention encoding the polypeptide of the invention is placed under the transcriptional control of regulatory signals functional in the cell. Said regulatory signals appropriately control the expression of the polypeptide of the invention to allow any necessary transcriptional and post transcriptional modification.

Preferably, the cell of the invention is a prokaryotic cell, more preferably a bacterial cell, even more preferably an *E. coli cell.*

Another aspect of the invention relates to the use of the cell of the invention for the production of the fusion polypeptide of the invention.

The cell of the invention may be cultivated for such purpose. A cell culture relates to the *in vitro* process of maintaining and growing cells. Cell cultures need controlled conditions of temperature, pH, percentages of gases (oxygen and carbon dioxide), in addition to the presence of the adequate nutrients to allow cellular viability and division. The skill in the art will know which conditions must be applied to the cell culture depending on the requirements of the selected cell. Cell cultures can be carried out in solid substrates, such as agar, or in a liquid medium, which enables the expansion of large amounts of cells in suspension.

Once the cell of the invention has been cultivated and the polypeptide of the invention has been expressed, it can be purified. The term "to purify", as used in the description, relates to the isolation of the polypeptide of the invention from the other polypeptides present in the culture medium in which the cell of the invention has grown. The isolation of the polypeptide can be carried out using differential solubility techniques, chromatography, electrophoresis or isoelectric focusing. Chromatography techniques can be based on molecular weight, ion charge (based on the ionisation state of the amino acids under working conditions), the affinity of the protein for certain matrixes or chromatographic columns, or by means of purification tags, and can be carried out on a column, on paper or on a plate. The isolation of the polypeptide can be carried out, for example, by means of precipitation with ammonium sulphate, fast protein liquid chromatography (FPLC) or high performance liquid chromatography (HPLC), using automated systems that significantly reduce purification time and increase purification efficiency.

Another aspect of the invention refers to an *in vitro* use of the fusion polypeptide, the nucleic acid sequence or the genetic construct of the invention for inducing the formation of cytotoxic amyloid oligomers in a cell, preferably in a prokaryotic cell, more preferably in a bacterial cell, even more preferably in a *E. coli* cell. Preferably, this use comprises exposing the cell to blue light. In this *in vitro* use, the cell is expressing the fusion polypeptide of the invention.

The term "in vitro" means the use as described above on cells growing in an *in vitro* culture or on cells that are outside of the human or animal body present, for instance but without limitation, in an *ex vivo* biofilm, in an *ex vivo* isolated biological sample (e,g., transcription-translation coupled systems such as PURE®), in an inert surface, in a scaffold, inside lipid vesicles (liposomes) or within a bioreactor.

"Blue light" is understood, in the context of this invention, as light emission intensity in the range of, preferably, between 1,000 Lux and 30,000 Lux. Blue light may be provided by, for instance but without limitation, a custom-built device made of 27 x 8 blue AlGaInP LED bulbs (5 mm ø, 20 deg cone, 3 V, λmax= 468 nm, 8 cd each), that allows regulation of the light emission intensity between 1,000 (min.) and 30,000 (max.) Lux, or any commercial blue (actinic) light LEDs arrays.

Since the polypeptide of the invention allows triggering the formation of cytotoxic amyloid oligomers within cells upon exposure to blue light, it can be used for killing undesired cells *ex vivo* (for instance, in a biofilm, bioreactor, surface or consortium) or undesired cells within organisms where they are inducing an infection or an undesired pathological condition.

Thus, another aspect of the invention refers to the use of the fusion polypeptide, the nucleic acid sequence, the genetic construct or the cell of the invention for eliminating or killing undesired cells *ex vivo,* more preferably cells present in a biofilm, bioreactor, surface or consortium.

In a preferred embodiment, the undesired cells are bacterial cells, more preferably *E. coli* or *S. aureus* cells, even more preferably *E. coli* cells.

The term "ex vivo" means outside of the human or animal body.

Another aspect of the invention refers to the use of the fusion polypeptide, the nucleic acid sequence, the genetic construct or the cell of the invention for:
- assembling amyloid nanoscaffolds useful, for instance but without limitations, for engaging enzymes in sequential reaction steps, or
- building transcriptional switches useful, for instance but without limitations, for synthetic gene expression circuits or light-controlled plasmid replication cassettes.

Another aspect of the invention refers to a pharmaceutical composition, hereinafter "the composition of the invention", comprising the polypeptide, the nucleic acid sequence, the genetic construct or the cell of the invention, preferably the polypeptide of the invention comprising, more preferably consisting of, SEQ ID NO: 3.

This composition of the invention comprises the polypeptide, the nucleic acid sequence, the genetic construct or the cell of the invention in a therapeutically effective amount. A "therapeutically effective amount" is understood to be the amount of polypeptide, the nucleic acid sequence, the genetic construct or the cell of the invention that, when administered to the patient, produces the desired effect, thereby triggering the formation of cytotoxic amyloid aggregates upon blue-light exposure and therefore killing the cell/s causing the pathological condition, preferably the infection, more preferably the bacterial infection. The therapeutically effective amount may vary depending on a variety of factors, for example, but not limited to, the type of pathological condition and its severity, as well as age, weight, sex, physical condition, response or tolerance, etc., of the individual to whom the composition of the invention is going to be administered.

Preferably, this composition of the invention further comprises a blue LED device or a blue LEDs light source for illuminating the cells comprising the polypeptide, the nucleic acid sequence or the genetic construct of the invention.

In a more preferred embodiment, the composition of the invention further comprises a pharmaceutically acceptable vehicle or excipient, adjuvant and/or other active ingredient.

Another aspect of the invention refers to the fusion polypeptide, the nucleic acid sequence, the genetic construct or the cell of the invention for use as a medicament. Alternatively, this aspect of the invention refers to the use of the fusion polypeptide, the nucleic acid sequence, the genetic construct or the cell of the invention for the manufacture of a medicament.

Preferably, the medicament of the invention is an antimicrobial medicament, more preferably an antibacterial medicament.

The term "medicament" or "drug" makes reference to any substance used to prevent, alleviate, treat or cure diseases, conditions or pathologies, preferably bacterial infections, more preferably E. *coli* infections, in humans, or in any other animal.

In the context of the present invention, the term "medicament" relates to a preparation that comprises the polypeptide, polynucleotide, gene construct or cell of the invention; preferably the polypeptide of the invention, more preferably the polypeptide of the invention comprising, even more preferably consisting of, SEQ ID NO: 3.

The medicament to which the present invention refers may be for human or veterinary use. The "medicament for human use" is any substance or combination of substances that have the properties for treating or preventing diseases in human beings or that can be used in human beings or administered to humans for the purpose of restoring, correcting or modifying physiological functions by exercising a pharmacological, immunological or metabolic action. The "medicament for veterinary use" is any substance or combination of substances having curative or preventive properties with respect to animal diseases or conditions or that can be administered to the animal in order to restore, correct or modify its physiological functions by exercising a pharmacological, immunological or metabolic action.

The medicament referred to in the present invention may be used together with other active ingredients or therapies in the manner of a combined therapy. The other active ingredients may form part of the same composition or can be provided by means of a different composition, being administered at the same time or at different times (simultaneous or sequential administration).

Another aspect of the invention refers to the fusion polypeptide, the nucleic acid sequence, the genetic construct or the cell of the invention for use in the treatment or prevention of microbial infections, preferably bacterial infections, more preferably E. *coli* infections. Alternatively, this aspect of the invention refers to the use of the fusion polypeptide, the nucleic acid sequence, the genetic construct or the cell of the invention for the manufacture of a medicament for the treatment or prevention of microbial infections, preferably bacterial infections, more preferably E. *coli* infections.

The "bacterial infections" may be produced by, for instance but without limitations, *E. coli* or common skin pathogens such as *S. aureus.*

Examples of microbial infections include, but without limitations, skin/dermatological infections, digestive infections or respiratory infections.

The term "treatment", as understood in the present invention, refers to combating the effects caused as a result of the disease or pathological condition of interest in an individual (preferably a mammal and, more preferably, a human), which includes:
(i) inhibiting the disease or pathological condition, i.e. interrupting its course;
(ii) alleviating the disease or pathological condition, i.e. causing the regression of the disease or pathological condition or its symptoms;
(iii) stabilising the disease or pathological condition.

The term "prevention", as understood in the present invention, consists of avoiding the appearance of the disease or pathological condition in an individual (preferably a mammal and, more preferably, a human), particularly when said individual is susceptible of developing the disease or pathological condition but has not been diagnosed yet.

Another aspect of the invention relates to a method, preferably an *in vitro* method, for inducing the formation of cytotoxic amyloid oligomers in a cell, preferably in a bacterial cell, which comprises:
a. expressing the fusion polypeptide of the invention in the undesired cell to be killed, and
b. exposing the cell of step (a) to blue light.

As explained above, the fusion polypeptide of the invention may be expressed in the cell by transfecting the nucleic acid sequence or the genetic construct of the invention, preferably through a bacteriophage, and placing the cell under suitable culture conditions that allow the expression of the polypeptide.

When the cells to be killed are within the human or animal body, the expression of the polypeptide of the invention in said cells may be achieved by the use of adequate expression vectors, preferably bacteriophages, that specifically direct the expression of the polypeptide to the interior of said cells.

The exposure of the cell to blue light, according to step (b) of the method, is preferably performed during at least 1h, but can extend longer and be applied either in a continuous regime or through light/darkness pulses of variable length.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### DESCRIPTION OF THE DRAWINGS

**Fig. 1****. Assessing the solubility of distinct helical phases for the Jα-α1 linker in LOV2-RepA-WH1 chimeras.** (**a**) Schematic representation of the arrangement of the three chimeras assayed. The linker helix increases its length by a helical turn between LOV540 (closest position related to WH1) and LOV543 (farthest to WH1), displaying both domains on opposite faces of the helix, whereas in LOV542 both are found on the same face and intermediate position. (**b**) Fractionation of bacterial cells (C) expressing the chimeras into the soluble (S) and insoluble (P) fractions (*top* panel), plus their detection by Western blotting (anti-His tag antibody; *bottom*)*.* Only LOV543-WH1 generates a major soluble fraction, with no major differences when cultures were carried out in the darkness or under blue light illumination. (**c**) These results are compatible with steric hindrance due to close apposition of the domains by a short linker (LOV540-WH1) and/or their mutual interference for folding (LOV542-WH1), whereas only LOV543-WH1 has the right distribution of both domains to fold independently.
**Fig. 2****. Limited proteolysis maps the Jα-α1 helical linker as the major light-altered structural element in LOV543-WH1.** (**a**) SDS-PAGE analysis of LOV543-WH1 digested, in the darkness or under blue light illumination, by three alternative proteases. A major cleavage site for chymotrypsin and V8 protease is made accessible in the illuminated state, generating two fragments with the sizes expected for LOV2 (≈17 kDa) and WH1 (≈15 kDa). (**b**) Sequence of the LOV543-WH1 fusion protein. Residue numbers are the originals from the AsLOV phototropin and RepA. N-terminal sequencing of the proteolytic fragments identified unambiguously the cleavage sites (arrows) for chymotrypsin (Ch: I₅₃₂KK and E₄₀₉RI) and V8 protease (V8: G₅₂₈VM). The N-terminal Met residue appears to have been removed *in vivo* (g-₂₃ss; tag peptide in lower case letters). Box outlines the linker Jα-α1 helix. The three amino acid substitutions included in the LOV543m3-WH1 (SEQ ID NO: 1) mutant are also indicated.

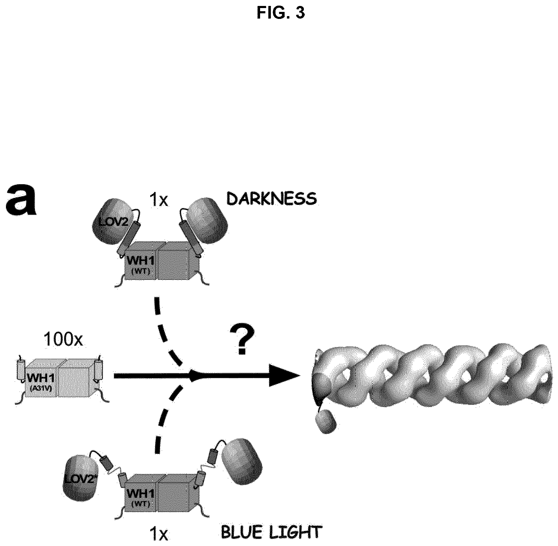

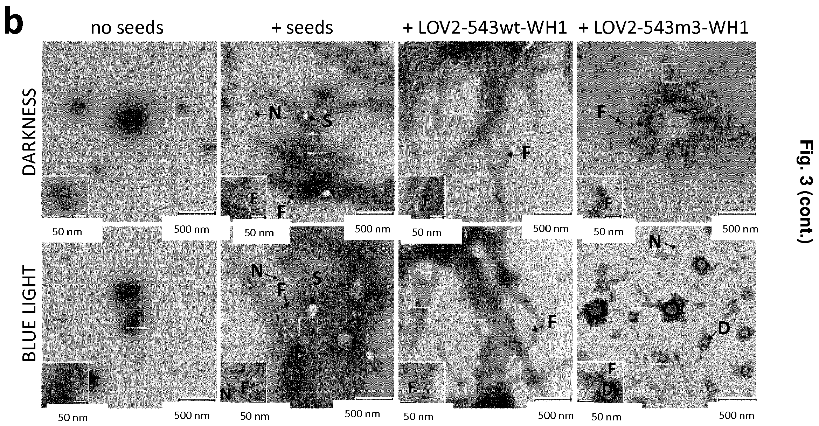

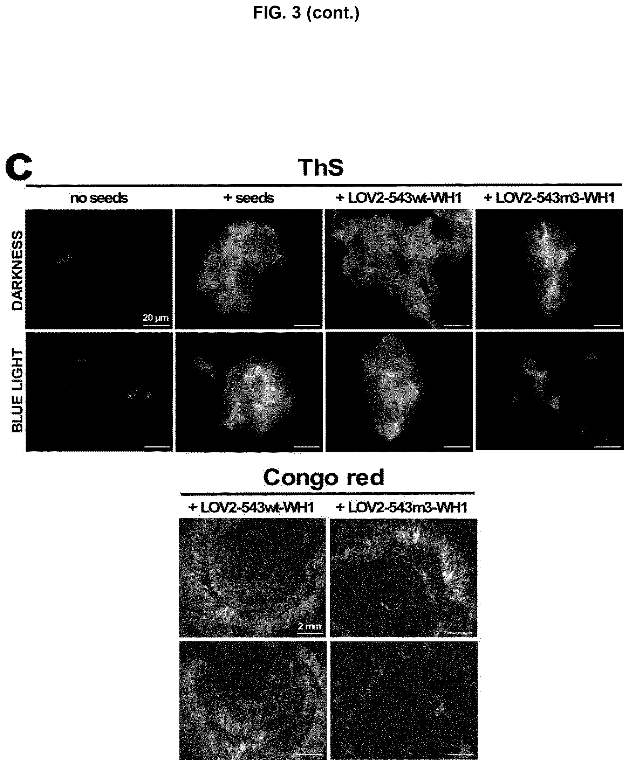

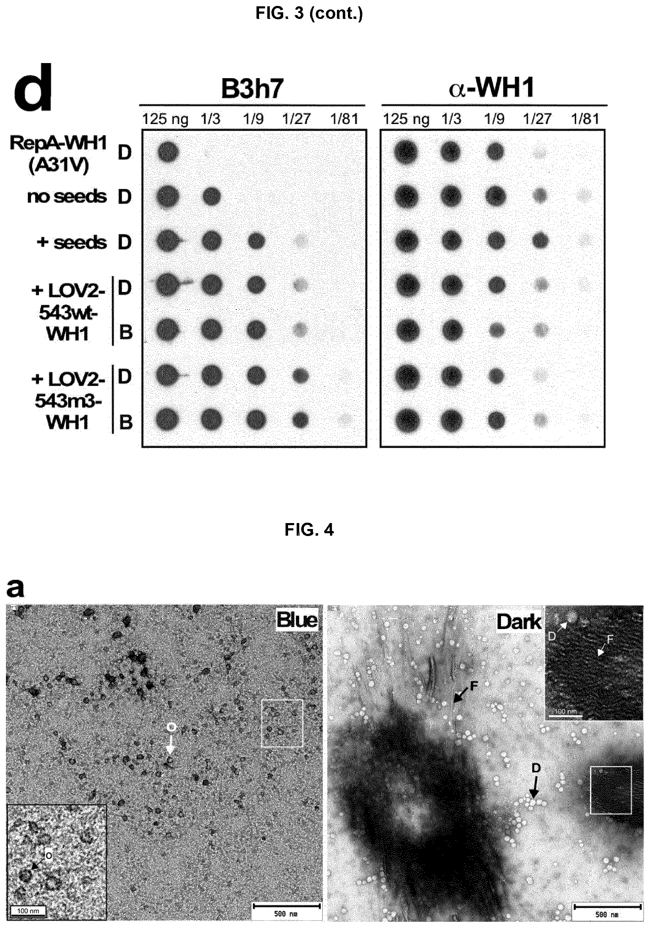

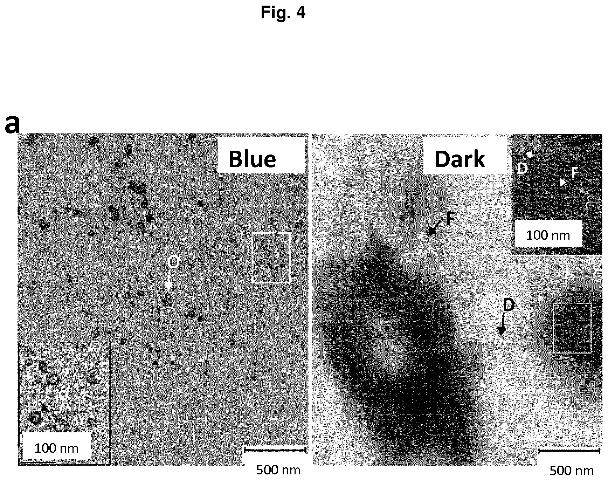

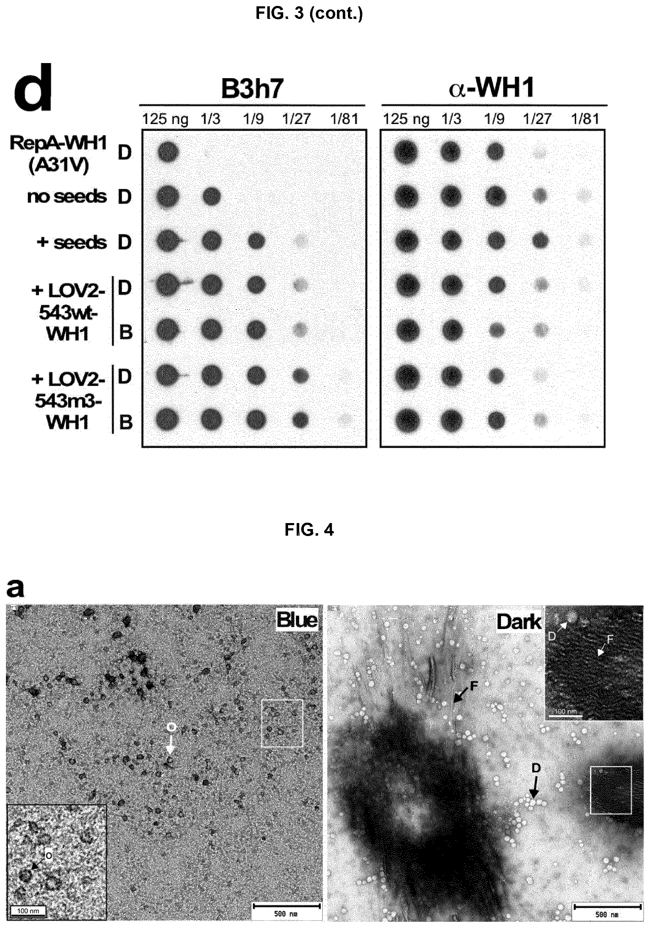

**Fig. 3****. *In vitro* cross-seeding of the assembly of the hyper-amyloidogenic protein RepA-WH1(A31V) (light grey) by sub-stoichiometric amounts (1:100) of the LOV543wt/m3-WH1 optogenetic switches.** (**a**) Schematic overview of the seeding experiment. (**b**) Electron micrographs of the aggregates generated during the incubation of RepA-WH1(A31V) as substrate and the indicated optogenetic seeds, either in the darkness or under blue light illumination. Control samples, un-seeded or adding as seeds RepA-WH1(A31V)-mCherry aggregates purified from *E. coli* (S), are also displayed. LOV543wt-WH1 templates on RepA-WH1(A31V) the assembly of large fibres (F), irrespective of being illuminated or not. LOV543m3-WH1 differentially seeds the growth of short fibres (darkness) or an ensemble of needle-shaped spikes (N) and spherical/drop-like oligomers (D) (blue light). Magnification: 20,000x (*insets:* 50,000x). (**c**) Analysis of the fluorescence emitted by ThS (*left*) and optical birefringence of Congo Red (CR, *right)* when incubated with samples in (a). Both amyloidotropic molecules bind to RepA-WH1(A31V) aggregates when templated by control seeds or by LOV543wt-WH1 (whether it was incubated in the darkness or under blue light), thus they are amyloids. Particles assembled by LOV543m3-WH1, especially when incubated under blue light, bind weaker to ThS and CR. (**d**) Dot-blot analyses. Serial dilutions of samples assembled in the darkness (D) or under blue light (B) are displayed. B3h7, an antibody specific for the conformation of amyloidogenic RepA-WH1 oligomers, shows that these are more abundant when seeded with LOV543m3-WH1 *(left).* A replica incubated with α-WH1, a polyclonal antibody against RepA-WH1, is included as a control (*right*). Overall, LOV543m3-WH1 generates an operational switch of amyloidogenesis, discriminating between the assembly of mature amyloid fibres (darkness) and amyloidogenic oligomeric particles (blue light).

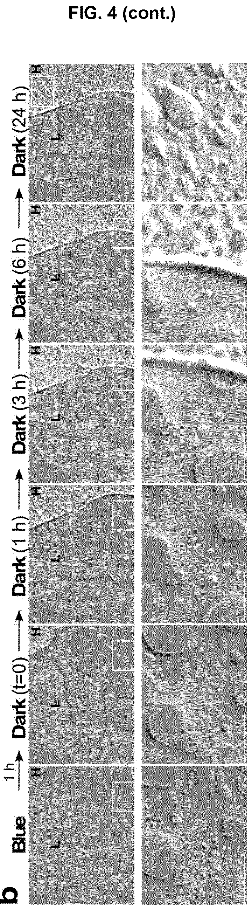

**Fig. 4****. Optogenetic regulation of a phase transition (liquid-hydrogel) in LOV543m3-WH1-mCherry.** (**a**) The hydrogel formed in the darkness, when examined by TEM, is made of a mixture of tightly packed fibres (F) and large spherical, drop-like oligomers (D), whereas the liquid formed under blue light contains discrete annular oligomers (o). Magnification: 20,000x (*insets:* 50,000x). (**b**) Epifluorescence visualization of a time course gelation experiment (*top row*). Samples were assembled under blue light and, after 1 h, transferred to the darkness for up to 24 h. Images are the superposition of DIC and TRICT (mCherry) channels. The boundary separating the liquid (L) and the expanding hydrogel (H) phases is clearly seen. Bottom row: zoom (5x) of the sectors boxed above.

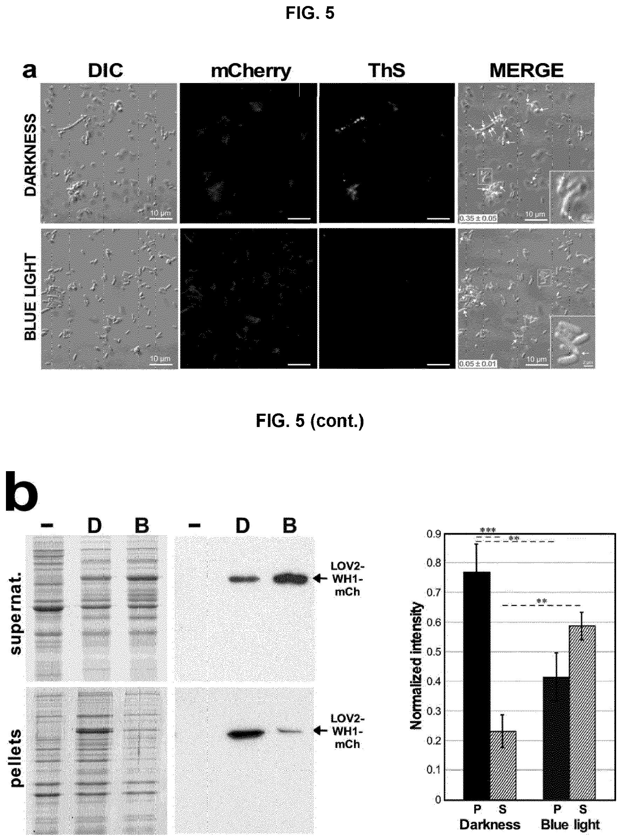

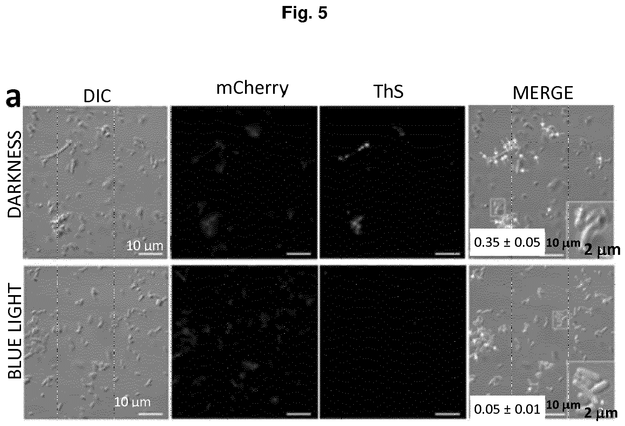

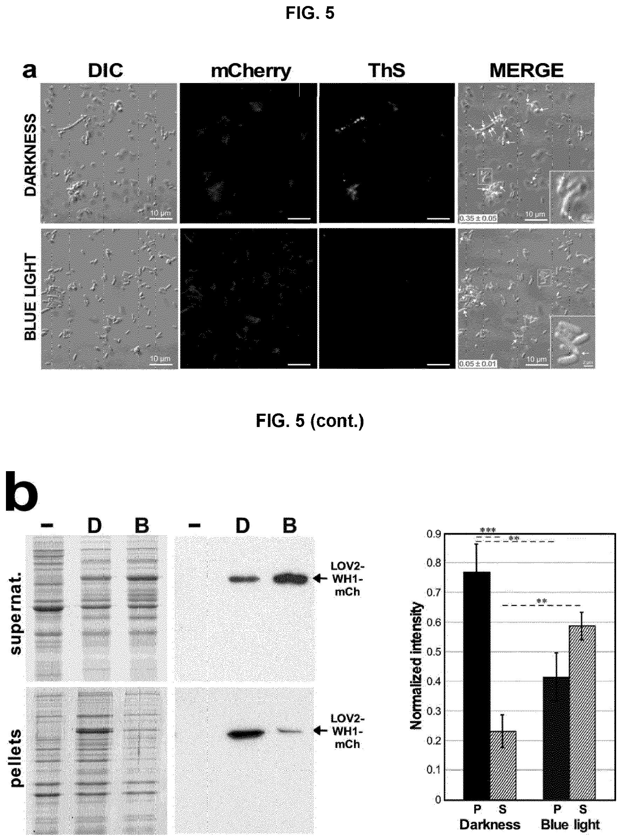

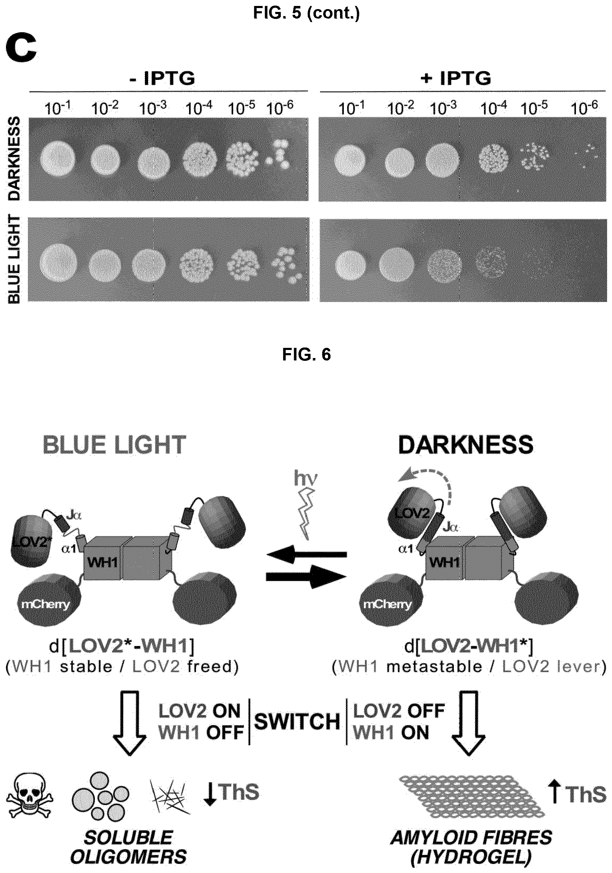

**Fig. 5****. Expression of LOV543m3-WH1-mCherry in *E. coli* results in the formation of large intracellular amyloid particles in the darkness, and smaller cytotoxic aggregates when grown under blue light.** (**a**) DIC and epifluorescence microscopies, showing the intrinsic mCherry fluorescence and the extrinsic ThS staining. Arrows point to intracellular aggregates. Experiment was independently repeated three times. 454 cells grown in the darkness and 1,534 under blue light were counted in total. (**b**) Fractionation of cell lysates of bacteria grown in (a). After centrifugation, proteins were analysed by SDS-PAGE (*left*) and then Western-blotted with an anti-mCherry antibody (*middle*). (-), non-induced cells control. P: pellets, S: supernatants. D: darkness, B: Blue light. Quantitation of the three culture replicas (one way ANOVA with Tukey's comparison test to 95% confidence; ***: p< 0.005, **: p< 0.05) indicated that blue light illumination during protein expression increases the solubility of the triple chimera. (**c**) Serial dilutions of cultures on agar plates show no significant inhibition of bacterial growth by blue light itself. Expression of LOV543m3-WH1-mCherry in the darkness results in much smaller colony sizes and in ≥10²-fold inhibition in the case of blue light illumination, an indication for cytotoxicity of the oligomers generated by the optogenetic switch when excited with blue light.
**Fig. 6****. Conformational pathways regulated by blue light in the LOV2-WH1 optogenetic device.** LOV2 allosterically selects between the assembly of the prion-like protein RepA-WH1 into large, non-cytotoxic hydrogels made of amyloid fibrils (right; darkness) and cytotoxic oligomers (left; blue light). In both scenarios, the Jα-α1 linker would act on the WH1 domain differentially, either as a rigid helical lever in amyloidogenic unfolding (darkness-promoted) or through the local destabilization of α1 (blue light-elicited).
**Fig. 7****. Site-directed mutagenesis to generate LOV543m3-WH1.** DNA sequencing chromatographic profiles for the wt and the m3 versions of the mutagenized stretch in LOV543-WH1.

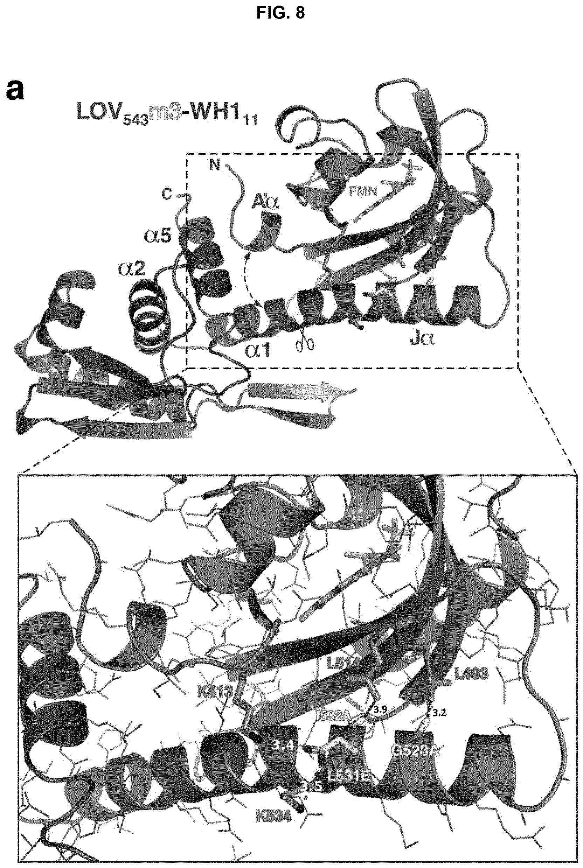

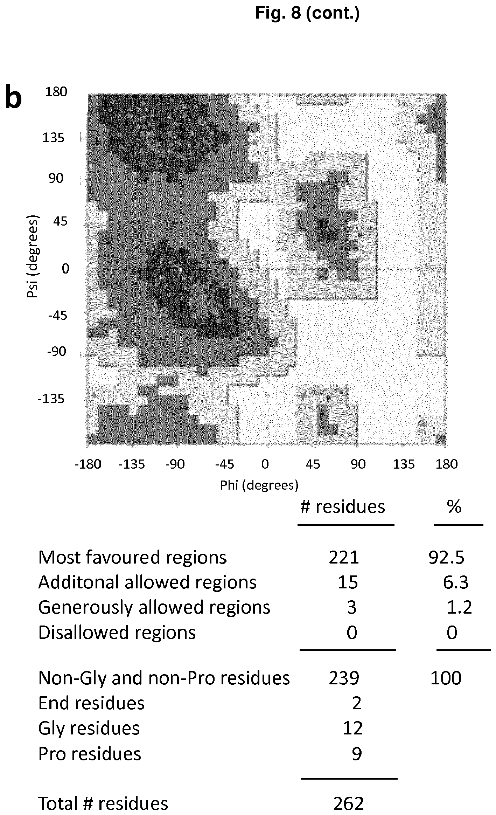

**Fig. 8****. Three-dimensional model of LOV543m3-WH1 in its dark state.** (**a**) Overview of a high score (0.86) LOV₅₄₃m3-WH1₁₁ model (*top*), as generated by the ROBETTA fully automated protein structure prediction server (Kim, D. E., Chivian, D. & Baker, D., 2004, Nucleic Acids Res., 32, W526-W531) on the structures of RepA-WH1 (1HKQ.pdb) and AsLOV2 in its dark state (2V0U.pdb). ROBETTA uses the ROSETTA fragment insertion method. Models were generated from structures detected by PSI-BLAST or HHSEARCH and aligned by HHSEARCH and SPARKS. Loops were built from fragments and optimized to fit the template structures. *Bottom:* Expanded view of the region of LOV543m3-WH1 comprising the chimeric Jα-α1 helix and its backing LOV2 β-sheet. Mutant side chains are displayed as sticks. (**b**) A Ramachandran plot, generated by PROCHECK, showing the stereochemical compatibility of the generated model. It is noteworthy that the first N-terminal residue modelled is Leu404 in LOV2, to which ROBETTA assigns the first position.

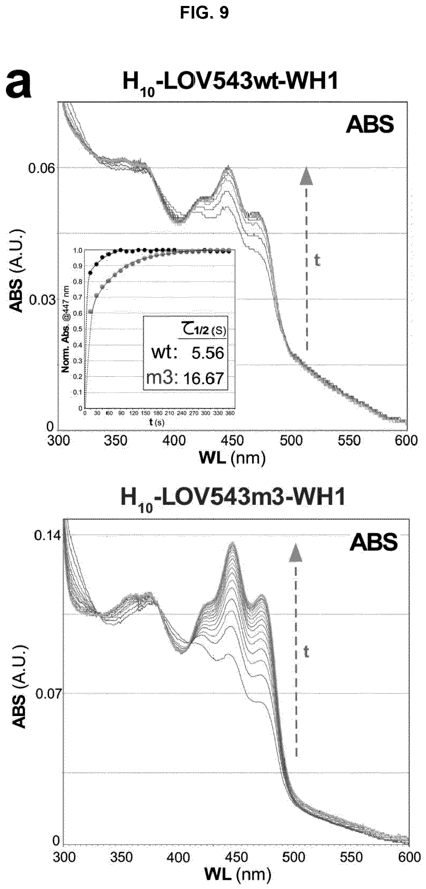

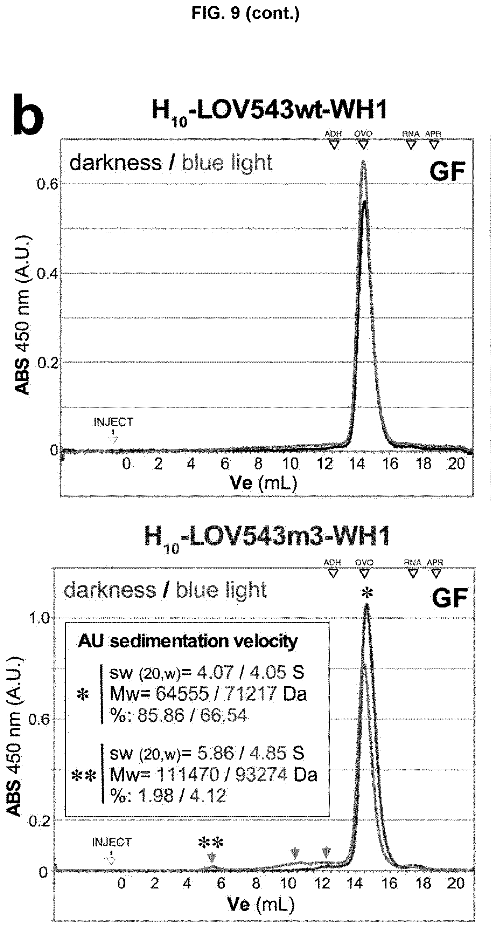

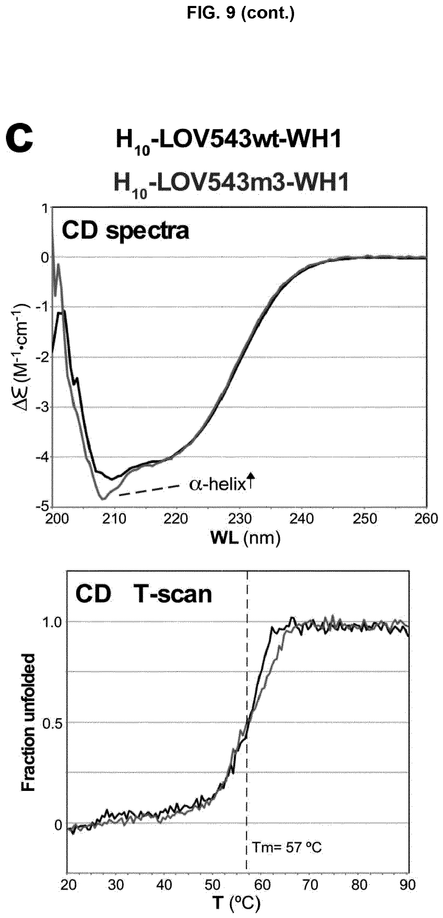

**Fig. 9****. Biophysical characterization of LOV543wt-WH1 *(left* column) and LOV543m3-WH1 (*right*).** (**a**) Absorption spectra showing a time course of the return of the blue light-excited chimeras to the ground (dark) state. Spectra were acquired with 15 s intervals after switching the blue LEDs off and then plotted overlaid. *Inset:* kinetics of the return to the ground state, measured as the value of the absorption band at 447 nm for each spectrum. Curves display double exponential function fittings to the data points. τ_{½}: half-life of the lit state. (**b**) Gel filtration elution profiles of the purified chimeras, run in the darkness or under continuous blue light illumination. The major species correspond to protein dimers, in spite of having been illuminated or not, as it was confirmed, for LOV543m3-WH1, through sedimentation velocity analyses (*inset*). Arrows point to minor oligomeric species. MW standards: aprotinin (APR), RNase A (RNA), ovoalbumin (OVO) and alcohol dehydrogenase (ADH). (**c**) CD spectra *(left)* and thermal denaturation profiles (*right*) of the purified chimeras.

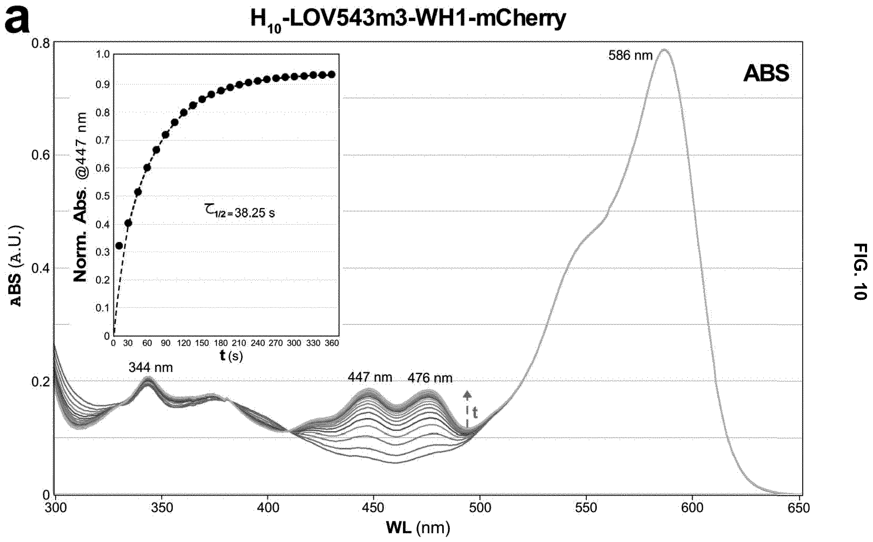

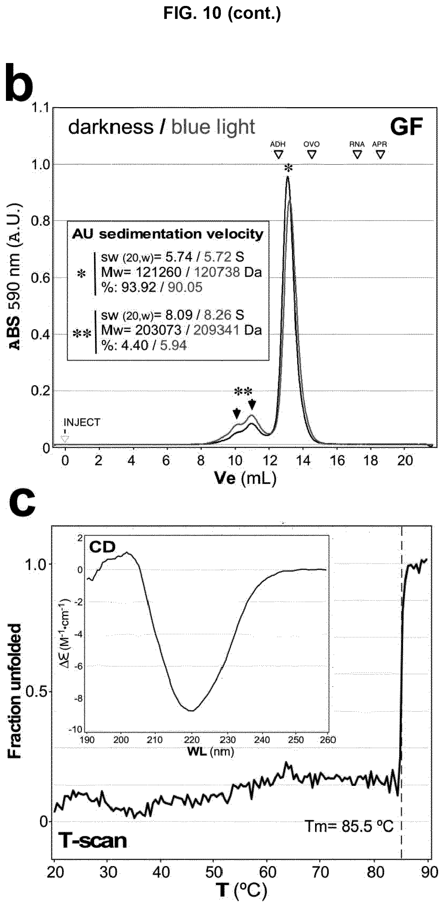

**Fig. 10****. Biophysical characterization of the LOV2m3-WH1-mCherry chimera.** (**a**) Absorption spectra displaying the return of the blue light-excited chimera to the ground (dark) state. Spectra were acquired with 15 s intervals after switching the blue LEDs off. *Inset:* kinetics of the recovery of the dark state, measured as the value of the absorption band at 447 nm for each spectrum. Curve shows a double exponential fitting for the data points. τ_{½}: half-life of the lit state. (**b**) Gel filtration elution profiles of the purified chimeras, run in the darkness or under continuous blue light illumination. The major species corresponds to a protein dimer, as confirmed by sedimentation velocity profiles (*inset;* single asterisk). Arrows and double asterisks point to minor oligomeric aggregated species. (**c**) CD thermal denaturation profile. *Inset:* CD spectrum.

### EXAMPLES

### Example 1. Results.

**LOV2-WH1 chimeras: design of a suitable helical linker.** The key determinant of RepA-WH1 (in short, WH1) stability is the formation of a helical latch by locking the C-terminal helix α5 in between the V-shaped N-terminal helices α1-α2. The possibility to manipulate the stability of WH1 by straining this domain at its N-terminus was explored constructing a chimeric continuous α-helix between the C-terminal Jα helix in the *Avena sativa* phototropin domain LOV2 and α1 in WH1. It is well established that upon absorption of blue light photons (λmax 447 nm) by the FMN chromophore in LOV2, Jα unfolds and detaches from the core of the domain, thus unconstraining the conformation of any sequence to which this helix had been intentionally linked. Three different helical phases in the Jα-α1 linker, and thus three distinct relative geometrical arrangements of the LOV2 and WH1, were constructed by PCR. The RepA-WH1 wild-type domain was used in the chimeras due to its higher solubility compared with some mutant variants (e.g., A31V, which is intrinsically hyper-amyloidogenic). The constructs (Fig. 1a) displayed both domains either at opposites sides (LOV540-WH1 and LOV543-WH1) or at the same side (LOV542-WH1) of the Jα-α1 helix. The difference between the former two chimeras is the length of the linker, being about a helical turn longer (4 amino acid residues) in LOV543-WH1 than in LOV540-WH1. The three chimeras were expressed in *Escherichia coli* with N-terminal Hisio tags, either in the darkness or under illumination with blue light (using a custom-built LED device) and, upon cell lysis, their sedimentation behaviour was tested (Fig. 1b). It turned out that only the LOV543-WH1 chimera differentially increased its solubility upon blue light illumination. The unresponsiveness to light of the other two constructs may well reflect either blocking of the switch by a too tight package of the domains, imposed by the reduced length of the linker (in LOV540-WH1), or steric hindrance by the N-terminal LOV2 domain to proper folding of the C-terminal WH1 (most likely in LOV542-WH1) (Fig. 1c). All subsequent experiments were thus performed with the LOV543-WH1 chimera.

The LED device used was made of 27 x 8 blue AlGaInP LED bulbs (5 mm ø, 20 deg cone, 3 V, λₘₐₓ= 468 nm, 8 cd each; TheLEDLight company), distributed in six independently switchable sectors and with a relay wheel that allows regulation of the light emission intensity between 1,070 (min.) and 30,000 (max.) Lux. These intensities were used, respectively, for the *in vivo* and the *in vitro* experimental settings.

**Addressing light-responsiveness of LOV2-WH1 through limited proteolysis.** Proteolysis is a useful test for the accessibility to the solvent of target peptide sequences, as well as for the stability of folded protein domains. Three of the specific proteases that were used to probe the full length RepA protein, thus uncovering the existence of its two tandem WH domains, were this time assayed on LOV543-WH1, either in the dark or under blue light illumination (Fig. 2a). Peptides were separated by SDS-PAGE, revealing that blue light enhanced the cleavage by chymotrypsin and V8 protease, which yielded two main protein bands whose sizes roughly corresponded to the expected for the individual LOV2 and WH1 domains. This suggested increased accessibility of the linker due to unfolding of the Jα-α1 helix upon blue light absorption. Then N-terminal peptide sequencing was performed on the chymotrypsin and V8 digestions that had been incubated under blue light, allowing for the identification of the major cleavage sites for each protease in the LOV543-WH1 sequence (Fig. 2b). Specific cleavage sites were found precisely at the joint between Jα-α1, confirming that the helical linker became unfolded, as designed, upon illumination of the LOV543-WH1 chimera with blue light.

**Improving the LOV2-WH1 switch by mutagenesis.** A major concern in the design of any synthetic switch through protein fusion is how this affects the dynamic range of the device, i.e., the net ratio between the response of a chimera to the ON and the OFF stimuli, which in optogenetics ultimately depends on the balance between the fraction of molecules that remain in the OFF (pseudo-dark) state upon illumination and the fraction of molecules that stay in the ON (pseudo-lit) state in the darkness. Three mutations were introduced in LOV543wt-WH1, to generate LOV543m3-WH1 (Fig. 7): I532A, G528A, and L531E, which improve the packing of the N-terminus of Jα with the backing β-sheet in LOV2, leading to a more tightly bound Jα helix in the dark state. The structure of the LOV543m3-WH1 chimera was modelled on the PDB coordinates of its two component domains using the Rosetta *de novo* structure prediction method. Fig. 8a displays a high-score *in silico* model supporting the structural feasibility of the three mutations within the fully folded LOV2 and WH1 frames, which showed no stereochemical violations (Fig. 8b).

The LOV543wt-WH1 and LOV543m3-WH1 chimeras were expressed in *E. coli,* purified and characterized through biophysical approaches (Fig. 9). Measurements of the return to the dark state after saturating blue-light stimulation, by following the evolution of the absorption spectra of the FMN prosthetic group (band at 447 nm), indicated a three-fold increase in the half-life of the excited state for the m3 mutant (16.7 s) compared with its parental wt protein (5.6 s) (Fig. 9a). No significant differences between the dark and lit sates, or between the wt and m3 chimeras, were observed regarding the association state of the proteins, as indicated by gel filtration analyses independently performed in the darkness and under blue light illumination (Fig. 9b). The higher solubility of the LOV543m3-WH1 chimera allowed confirmation of its dimeric state by sedimentation velocity analysis performed under both illumination conditions (Fig. 9b, *right, inset*). This observation suggests that, in the chimeras, dimerization continues to be dictated by the stable antiparallel β-sheet interface in RepA-WH1. Strikingly, LOV543wt-WH1 aggregated massively on any attempt to either freeze or concentrate it further than 6 µM, while LOV543m3-WH1 withstood both storage in a frozen state and at least 10-fold higher concentrations. Circular dichroism spectra of both chimeras in the darkness showed the typical α-helical profile, with a more pronounced band at 208 nm for the m3 chimera (Fig. 9c, *left*). This is a signature for increased α-helical content, as expected from the stabilization of Jα in the dark state by the three mutations engineered in LOV543m3-WH1. Thermal denaturation analyses (Fig. 9c, *left*) indicated that both chimeras were stable, showing a single transition between two states (folded and unfolded) with a Tm (50%) value of 57 ºC (Fig. 9c, *right).*

**Light modulates the capacity of LOV2-WH1 to cross-seed RepA-WH1(A31V) amyloidogenesis.** Seeding, i.e., the ability of a pre-formed amyloid aggregate to template and nucleate amyloid growth from soluble molecules of the same (or a closely related) protein, is a hallmark of amyloidogenesis. To test the capacity of LOV543-WH1 to act as a light-controlled switch in amyloid nucleation, substoichometric amounts of the purified chimeras (either the wt or m3 variant) were supplied to an excess of soluble RepA-WH1(A31V) (Fig. 3a). This is a hyper-amyloidogenic RepA-WH1 variant that efficiently assembles as fibres under standard *in vitro* conditions provided that a nucleation agent, such as purified RepA-WH1(A31V) aggregates preformed *in vivo,* are supplied as seeds. The formation of RepA-WH1(A31V) fibres was thus explored either under continuous blue light illumination or in the darkness. Fibrillation was monitored by transmission electron microscopy (TEM) (Fig. 3b), thioflavin-S (ThS) fluorescence emission, Congo red (CR) birefringence under polarized light (Fig. 3c) and probing with B3h7, a monoclonal antibody specific for oligomers of RepA-WH1 with an amyloidogenic conformation, but showing reduced affinity for the fibres (Fig. 3d). The results of these assays indicated that just spare amorphous aggregates were found in the absence of any supplied seed, while fibres were the product of nucleation by the intracellular RepA-WH1 (A31V) aggregates. Interestingly, similar fibrilar aggregates were generated upon the addition of LOV543-WH1 in its wt version. However, no differential fibrillation response to the darkness/blue light regimes was observed for this chimera. On the contrary, the LOV543-WH1m3 variant nucleated on RepA-WH1 (A31V) the formation of defined, short fibrils in the darkness, but of drop-like oligomeric particles and needles under blue light (Fig. 3b). These oligomeric species showed much reduced amyloid character, when compared to the mature fibres, according to both ThS and CR staining (Fig. 3c). The higher affinity of the B3h7 antibody in dot-blot assays for the reactions seeded with LOV543m3-WH1 supports their enrichment in amyloidogenic RepA-WH1 oligomers (Fig. 3d). Because only the LOV543m3-WH1 chimera was able to modulate the fibrilar/oligomeric assembly of RepA-WH1 *in vitro* under switch conditions (i.e., darkness/blue light), this protein fusion was selected to explore the capability to build by itself complex supramolecular assemblies, both *in vitro* and *in vivo.*

**LOV543m3-WH1-mCherry enables optogenetic control on phase transitions.** To visually follow the effect of darkness/blue light illumination on LOV543m3-WH1 aggregation, both *in vitro* and *in vivo,* the red fluorescent reporter protein mCherry was fused to the C-terminus of the WH1 domain. The LOV543m3-WH1-mCherry protein was then purified and characterized through several biophysical approaches (Fig. 10). The half-life of the excited state, measured as the recovery of the absorption band at 447 nm after transferring pre-illuminated samples to the darkness, was improved by 2.3-fold (to 38.3 s) compared to the LOV543m3-WH1 fusion, with the mCherry band (at 586 nm) dominating the spectra and showing no temporal variation (Fig. 10a). As it was shown for its parental double chimera (Fig. 9b, *right*), according to gel filtration and sedimentation velocity analyses, LOV543m3-WH1-mCherry remained as a dimer either in the darkness or under blue light (Fig. 10b). The CD spectrum (Fig. 10c) was much dominated by the β-sheet structure of mCherry, and the triple chimera showed enhanced thermal stability (85.5 ºC) compared to the double chimera (57 ºC; Fig. 9c, *right*). However, as for the LOV543wt/m3-WH1 fusions, it is noteworthy that these assays were carried out under ideal (diluted) buffer conditions, not attempting to mimic the crowded environment of the bacterial cytoplasm.

The assembly potential of LOV543m3-WH1-mCherry either in the darkness or under blue light illumination was then tested *in vitro* at a very high protein concentration (0.25 mM), in a low salt buffer and in the presence of polyethylene glycol (PEG) 4000, a crowding agent that enhances RepA-WH1 fibrillation (Fig. 4). In the darkness, the triple chimera decanted to the bottom of the test tubes with the visual appearance of a hydrogel, which clearly separated from the buffer supernatant. TEM revealed the coexistence within the hydrogel of spherical particles and tightly packed fibril bundles (Fig. 4a). On the contrary, when samples were illuminated with blue light, they stayed as a translucent red solution, in which just ring-like oligomers were evident by TEM (Fig. 4a). Experiments carried out to explore such phase transitions under an epifluorescence microscope revealed that samples remained liquid as long as they were illuminated with blue light, with some de-mixing into drops containing LOV543m3-WH1-mCherry. However, when turned into the darkness these drops readily fused and the protein solution evolved within hours into a thick hydrogel with the appearance of a sponge (Fig. 4b). As expected for amyloidogenesis, darkness-promoted gelation of LOV543m3-WH1-mCherry was not reversible.

**LOV543m3-WH1-mCherry is an optogenetic switch for bacterial proliferation.** Expression in the darkness of the LOV543m3-WH1 (WT)-mCherry triple chimera resulted in filamentation of bacterial cells (Fig. 5a), a phenotype characteristic of RepA-WH1 (A31V)-mCherry expression but not of RepA-WH1 (WT)-mCherry, suggesting that LOV2 indeed destabilizes the fold of the native RepA-WH1 turning it into amyloidogenic. Although quantitation is difficult due to clumping of a substantial fraction of the cells, 35% of them exhibited intense ThS-stainable protein aggregates, an indication for their amyloid nature. On the contrary, if expression of LOV543m3-WH1-mCherry was carried out under blue light illumination, just 5% of the cells showed ThS fluorescence emission, and this was of much lower intensity than when bacteria were cultured in the darkness. Biochemical analysis of the solubility of the triple chimera upon cell lysis and subsequent sedimentation plus Western-blotting (Fig. 5b) confirmed that the fraction of LOV543m3-WH1-mCherry in the supernatant increased in cultures illuminated with blue light (59%) compared with those grown in the darkness (23%).

To survey a possible effect of the optogenetically-regulated expression of LOV543m3-WH1-mCherry on bacterial proliferation, serial dilutions of exponential phase cultures that had been grown in the darkness were plated on LB-agar including (or not) the inducer IPTG (Fig. 5c). While in the absence of expression of the triple chimera no difference was appreciated between drops incubated in the darkness or under blue light illumination, LOV543m3-WH1-mCherry expression reduced the growth of bacteria under blue light by a least two logs when compared to dark-state incubation conditions. Since under blue light illumination both LOV543m3-WH1 (Fig. 3) and LOV543m3-WH1-mCherry (Fig. 4) form amyloidogenic oligomers *in vitro,* and RepA-WH1(A31V)-mCherry oligomers are the most cytotoxic molecular species of *in vivo,* oligomerization of the triple chimera likely is the basis for the observed decrease in *E. coli* viability upon blue light illumination.

In summary, the LOV2-WH1 chimeras were first optimized by modulating the phase and length of the linker Jα-α1 helix (LOV₅₄₃-WH1₁₁; Fig. 1), plus the inclusion of mutations to stabilize the dark state conformation of Jα (Figs. 7-9). *In vitro* studies (Fig. 3) suggest that, in the darkness, the stiff Jα-α1 chimeric helix connecting LOV2 and WH1 (Fig. 2) would act as a lever that, by unleashing the three-helix bundle (α1-α2-α5) that locks the fold of WH1, would enable the protein to template, on a hyper-amyloidogenic RepA-WH1 variant, the assembly of amyloid fibrils (Fig. 6a, *right*). On the contrary, under blue light illumination the partial unfolding of the Jα-α1 linker (Fig. 2) would compromise the α1-α2-α5 latch in a milder way, resulting in a conformational ensemble that templates the assembly of distinct oligomers (Fig. 3), either needle-shaped or drop-like, that show a weaker amyloid nature (Fig. 6a, *left*). Such optogenetic destabilization of the N-terminus of RepA-WH1 is a new way to trigger amyloidogenesis in this protein.

Therefore, light-modulated conformational remodelling of the LOV543m3-WH1 chimeras is a reliable approach to gain control on RepA-WH1 amyloidogenesis and toxicity, either *in vitro* or *in vivo.* Here it is shown that an optogenetic switch achieving control over protein amyloidosis, both *in vitro* and *in vivo,* can be built by combining in a chimera a light-responsive LOV2 domain with a versatile amyloidogenic bacterial module, RepA-WH1.

Amyloid opto(epi)genetics remained yet unexplored. The LOV2-WH1 chimeras presented herein enrich and expand the catalogue of available optogenetic tools with a novel way to guide the conformational landscape of proteins towards amyloidogenesis. Their potential applications may include controlling the assembly of amyloid nanoscaffolds to engage enzymes in sequential reaction steps; building transcriptional switches for synthetic gene expression circuits and light-controlled plasmid replication cassettes; the selective elimination of particular bacteria within a consortium, once they have fulfilled their task in a bioprocess; or the development of a completely new kind of antimicrobials based on triggering amyloidosis by, e.g., bacteriophages encoding light-switchable cytotoxic amyloids.

### Example 2. Methods.

### Construction of the LOV2-WH1 chimeras

The *AsLOV2* gene (SEQ ID NO: 6) was custom-synthesized at ATG:biosynthetics (Merzhausen, Germany), with its codon composition optimized to the usage in *E. coli* (SEQ ID NO: 7), and delivered as a pUC18 derivative. The template source of *repA-WH1* was pWH1(WT). Both genes were independently amplified by PCR, using *Pfu* DNA polymerase, in such a way that the primers at 3' end of *LOV2* and at the 5'end of *repA-WH1* hybridize in the next step through their 5' ends. Three alternative pairs of these linker primers were designed to generate three distinct transitions in the fusion between both domains: LOV₅₄₀-WH1₁₂, LOV₅₄₂-WH1₁₂ and LOV₅₄₃-WH1₁₁. A second PCR round on an equimolar mixture of both amplicons, and with the primers annealing at the 5' end of *LOV2* and the 3' end of *repA-WH1,* yielded the three distinctly phased chimeras. The amplified chimeric fragments were then cloned into pRG-Ptac-His₁₀-*ORC4*, by replacing the resident *ORC4* gene through restriction with Sacll and HindIII plus ligation (T4 DNA ligase).

For the site-directed mutagenesis to generate LOV543m3-WH1 the following oligonucleotides were used: G528A_I532A-F (SEQ ID NO: 8): 5' GTGATGCGGCGGAACGTGAAGCCGTGATGCTGGCTAAAAAAACCGCAG AAAACATTGAT and G528A_I532A-R (SEQ ID NO: 9): 5' ATCAATGTTTTCTGCGGTTTTTTTAGCCAGCATCACGGCTTCACGTTCCG CCGCATCAC, to build the G528A and I532A double mutant. On this double mutant the following primers were used to build the triple mutant (including the L531E mutation): G528A_L531E_I532A-F (SEQ ID NO: 10): 5' CGGAACGTGAAGCCGTGATGGAGGCTAAAAAAACCGCAGAAAACA and G528A_L531E_I532A-R (SEQ ID NO: 11): 5' TGTTTTCTGCGGTTTTTTTAGCCTCCATCACGGCTTCACGTTCCG.

The LOV2m3-WH1-mCherry chimera was built in an analogous way, but using pRG-Ptac-His₁₀-L*OV543m3-WH1* (see above) and *mCherry* (from pRG-Ptac-His₆-*mCherry*) as the templates for the two PCR amplifications at the initial step. The product of the second PCR amplification round, once digested with Spel and Hindlll, was cloned into pRK2-Ptac-His₁₀+*lacI^{q}*, a derivative of the low copy-number vector pSEVA121. All constructs were verified through DNA sequencing (Secugen, Madrid).

### Protein expression and purification

The RepA-WH1(A31V) protein used in the fibrillation studies was purified as described (Giraldo, R., 2007, Proc. Natl. Acad. Sci. USA, 104, 17388-17393). The LOV2-WH1 chimeras (Hio-LOV543wt/m3-WH1 and H₁₀-LOV543m3-WH1-mCherry) were expressed in the *E. coli* strain BL21, in the presence of a helper plasmid providing T7 lysozyme to facilitate cell lysis. 0.75 L of Terrific Broth medium supplemented with ampicillin (Ap) to 100 µg·mL⁻¹ was inoculated with colonies from overnight LB agar plates with Ap₁₀₀ and chloramphenicol (Cm) to 30 µg·mL⁻¹ and grown at 37 ºC to an OD₆₀₀ₙₘ ≈ 0.8. Then, IPTG was supplied to 1.0 mM and the flasks covered with aluminium foil. Expression proceeded for 5 h at room temperature (RT). Cells were harvested, washed with cold 0.9 NaCl and resuspended in 15 mL of lysis buffer (0.5 M NaCl, 0.05 M imidazole pH 8.0, 1% Brij-58, 10% glycerol plus 1 pill of EDTA-free Roche protease inhibitors). Cell suspension was frozen at - 70 ºC.

Cell lysis was enabled by thawing the cell suspension to RT and a clarified lysate was obtained by ultracentrifugation at 62,000 x g for 1 h at 4 ºC. Supernatant was distributed in two aliquots and each one was independently loaded into an ABT Ni-affinity 5 mL cartridge wrapped in aluminium foil and coupled to an ÄKTA basic 10 FPLC (GE Healthcare). After an extensive wash with column buffer A (0.5 M NaCl, 0.05 M imidazole pH 7.8, 10% glycerol), a 25 mL linear gradient was run between this buffer and column buffer B (0.5 M NaCl, 0.75 M imidazole pH 7.8, 10% glycerol). Peak fractions were pooled and stored at 4 ºC. Further purification plus buffer exchange, to eliminate imidazole which inhibits the transition of LOV2 to the lit state, was achieved by size-exclusion chromatography (SEC) in a Superdex HR-200 column (GE Healthcare) equilibrated and run at 0.4 mL·min⁻¹ flow in SEC buffer (0.05 M Na₂SO₄, 0.010 M Hepes·NaOH pH 7.6, 0.1 mM EDTA). Peak elution profiles were monitored at A₂₈₀, A₄₄₇ and (for the chimera including mCherry) A₅₉₀ nm.

The concentration of the purified proteins was determined by absorption at 280 (RepA-WH; ε= 11,548 M⁻¹·cm⁻¹), 447 (H₁₀-LOV543wt/m3-WH1; ε= 13,800 M⁻¹·cm⁻¹) or 590 (H₁₀-LOV543m3-WH1-mCherry; ε= 70,700 M⁻¹·cm⁻¹) nm. Protein chimeras were stored at 4 ºC in the darkness for up to a week.

### Protein solubility assays

Solubility of the chimeras, expressed in the *E. coli* K-12 reduced genome strain MDS42, was assayed in whole cell lysates from 15 mL of cultures grown at 37 ºC in LB plus Ap₁₀₀. When bacterial cultures reached OD₆₀₀ₙₘ= 0.2, IPTG was added to 0.5 mM and they were split into two aliquots, to be grown either in the darkness or under blue light illumination (1,070 Lux). After 4 h of induction cells were harvested and resuspended in 0.2x lysis buffer (see above), at a ratio of 0.33 mL per each unit of optical density (1.5 x 10⁹ cells). EDTA (to 1 mM) and lysozyme (to 1 µ·mL⁻¹) were supplemented and incubation proceeded for 15 min at RT. Cell lysates were centrifuged at 16,100 x g for 1 h at 4 ºC and the supernatant and pellet fractions were carefully separated.

Both fractions were then analysed by SDS-PAGE (12.5% polyacrylamide gels), loading equal volumes of each supernatant and its corresponding resuspended pellet. Samples were run in duplicate: one set for Coomassie blue staining (whole protein detection) and the other for Western blotting. Transference to PVDF membranes was carried out by semi-dry blotting, followed by blocking in TTBS plus powder milk. Primary antibodies were used at 1:20,000 dilution, either mouse anti-His (Sigma) or rabbit anti-mCherry (Abcam), then incubated with HRP-conjugated secondary antimouse/rabbit antibodies at 1:20,000 (Sigma). Antibody binding was detected using the ECL 2 substrate (Pierce-Thermo) and X-ray films (AGFA Curix RP2 plus).

### Limited proteolysis

Three µg aliquots of purified Hio-LOV543wt-WH1 were displayed in 15µL of SEC buffer and trypsin (0.025 units), chymotrypsin (0.004 u.) or V8 (0.05 u.) proteases (Sigma) were supplied on ice. Digestions were left to proceed, either in the darkness or under blue light illumination (30,000 Lux), for 1 and 2 h at RT. Reactions were stopped by adding SDS-PAGE loading buffer and immediately boiling for 5 min, followed by electrophoretic separation in 12.5% polyacrylamide gels and Coomassie blue staining. Replicated samples of the chymotrypsin and V8 digestions (1 h, blue light) were transferred frozen to the Protein Chemistry facility at CIB-CSIC for Edman's N-terminal sequencing (5 cycles in a Procise 494 sequencer, Applied Biosystems).

### Optogenetic seeding of RepA-WH1(A31V) amyloidogenesis in vitro

Amyloidogenesis assays *in vitro* were carried out as described (Molina-Garcia, L., et al., 2018, Methods Mol. Biol., 1779, 289-312), by setting on ice in 2 mL Eppendorf tubes 50 µL aliquots made of: RepA-WH1(A31V) (25µM) in fibril assembly buffer (0.1M Na₂SO₄, 4 mM MgSO₄, 20 mM Hepes·NaOH pH 8.0, 14% PEG4000, 6% MPD), but using as seeds sub-stoichiometric amounts (1:100) of the purified Hio-LOV543wt/m3-WH1 chimeras. As controls, both un-seeded samples and others including RepA-WH1(A31V)-mCherry aggregates (0.1 µg) as seeds were also casted. Samples were then incubated under continuous shaking, at 300 rpm and 25 ºC for 3 h, in a thermomixer (Eppendorf), either in the darkness or illuminating with blue light (30,000 Lux). The tubes were pre-covered with aluminium foil or a thin PVC film, respectively, to avoid or allow the passage of light while preventing evaporation.

Two µL aliquots of the samples were then immediately diluted (1:10) in water, blotted to carbon-coated copper grids (400 mesh; EM Sciences) for negative staining with 2% uranyl acetate and subsequent visualization in a JEOL JEM-1230 electron microscope operated at 100 kV.

For amyloid detection with amyloidotropic molecules, five µL aliquots of the samples were incubated with 200 µL of a thioflavin-S (ThS) solution (0.05% w/v in 12.5 ethanol) for 30 min at RT. The suspension was then centrifuged (16,100 x g for 1 h at 4 °C) and the pellets washed twice with 200 µL of PBS buffer. Final aggregates were resuspended gently in 5 µL PBS and displayed on glass slides until drying. Samples were inspected in a Nikon Eclipse 90i epifluorescence microscope using 40x Plan Fluor objective (NA= 0.75) and a FITC filter (λₑₓ= 482/35; λₑₘ= 536/40; exposure: 1 s). In parallel, 2 µL aliquots were displayed on glass slides, left to dry and incubated with 2µL of a saturated solution of Congo red (CR) in 70% ethanol. Samples were then inspected for green-apple birefringence in a stereomicroscope (Leica MZ12₅) working at 3.2x magnification and with its two linear polarizers crossed at 90º. For immunodetection of amyloidogenic protein species, two µL sample aliquots were also serially diluted in fibril assembly buffer, but with no PEG4000 or MPD added, and spotted on a nitrocellulose membrane (Hybond-C extra; GE Healthcare) casted in a Bio-Dot vacuum-blotter (Bio-Rad). Membranes were then blocked as for Western blots (see above) and incubated with B3h7, a mouse conformational antibody specific for amyloidogenic oligomers of RepA-WH1 (1:5,000), or the rabbit polyclonal antibody α-WH1 (1:1,000). Secondary antibodies incubations and luminescence detection were performed as above.

### Assessing H₁₀-LOV543m3-WH1-mCherry phase transitions (liquid-hydrogel) in vitro

40 µL samples were assembled in Eppendorf tubes under blue light (30,000 Lux), including LOV543m3-WH1-mCherry (250 µM) in 0.5x SEC buffer, 0.008% ThS (see above), 9% PEG4000 and 4% MPD. After 2 h illumination, pictures were taken and the tubes were transferred to the darkness and incubated overnight at room temperature before taking a second set of photos. Two µL of each type of samples were picked-up by pipetting, stained with uranyl acetate and observed by TEM (see above).

In parallel, 10 µL drops with identical composition were casted on glass slides under blue light illumination, and then immediately layered with a cover slip. Specimens, sealed with nail polish to avoid drying, were kept under blue light illumination at the microscope setting for 1 h, and then light was switched-off and incubation proceeded for up to 24 h at room temperature. Epifluorescence was examined at the indicated time intervals in a Nikon Eclipse 90i microscope, using a 60x Plan Apo oil immersion objective (NA= 0.95) and TRITC (λₑₓ= 543/22; λₑₘ= 593/40; exposure: 0.5 s) and cyan (λₑₓ= 438/24; λₑₘ= 483/32; exp.: 0.3 s) filters. Differential interference contrast (DIC) images were also acquired (exp.: 0.3 s).

### Optogenetic switching of H₁₀-LOV543m3-WH1-mCherry amyloidogenesis in vivo

*E. coli* MDS42 cultures carrying the *pRK2-LOV543m3-WH1-mCherry* plasmid were grown in 100 mL of LB medium plus Ap₁₀₀, in the darkness at 37 ºC, to OD₆₀₀ₙₘ= 0.2, when cultures were split into two 45 mL aliquots, then placed in sterile bottles that included a magnetic bar. The rest of the cultures were left to grow to OD₆₀₀ₙₘ= 1.0, when serial dilutions (7 µL drops) were spotted on LB-agar plus Ap₁₀₀ +/- 1.0 mM IPTG for testing viability of bacteria, upon incubation with blue light (1,070 Lux) or in the darkness, for 30 h at 37 ºC. To the two 45 mL culture aliquots, IPTG was added (to 0.5 mM) and one of the flasks was covered with aluminium foil while the other was left unwrapped. Incubation under blue light (as above) or in the darkness proceeded at 37 ºC with stirring (150 rpm) for up to 4 h. Cells from 15 mL of the cultures were harvested and washed twice with PBS. Pellets were resuspended in 1 mL of PBS and, while 0.9 mL were centrifuged and the cells stored at -70 ºC for solubility tests (see above), bacteria from the other 0.1 mL were fixed with 4% paraformaldehyde (Sigma) for microscopy. Fixed cells were then stained with ThS, as indicated above. Bacteria were observed in a Nikon Eclipse 90i microscope, as in the previous section, but using a 100x Plan Apo oil immersion objective (NA= 1.4).

### Biophysical characterization of the H₁₀-LOV543wt/m3-WH1 and H₁₀-LOV543m3-WH1-mCherry chimeras

Purified protein chimeras were characterized for their response to darkness and blue light illumination regarding their photocycle, association state, secondary structure and stability in SEC buffer.

The photocycle of the LOV2 moiety in the H₁₀-LOV543wt/m3-WH1 chimeras was studied by saturating with blue LEDs illumination (30,000 Lux, for 10 min) 600 µL protein solutions (3 µM wt and 5 µM m3) that were displayed in quartz cuvettes (1 cm path length) placed into the sample holder of an Ultrospec 3300pro spectrophotometer (GE Healthcare). To measure the return of excited flavin chromophore to the dark state, immediately after switching the light off time-lapsed spectra acquisition started under the control of the Swift II software, with the following parameters: 300-600 nm wavelength interval (0.5 nm/data point); 2,649 nm/min scan speed; 24 accumulated spectra (i.e., one every 15 s). For the H₁₀-LOV543m3-WH1-mCherry chimera (8 µM), wavelength acquisition interval was extended to 650 nm to get the full band from excitation of mCherry. Return to dark state was analysed by fitting (MATLAB, The MathWorks Inc., release 2010a) a biexponential Levenberg-Marquardt function (R²= 0.9941 wt, 0.9771 m3, 0.9929 mCherry) to the A₄₄₇ₙₘ data points, corresponding to the main dark absorption band in LOV2.

For determining the association state of the chimeras, besides SEC (100 µL samples, 50 µM; see above), sedimentation velocity experiments were performed in a Beckman-Coulter Optima XL-I analytical ultracentrifuge, at 48,000 rpm and 20 ºC, with a protein concentration (in SEC buffer) of 5 µM (H₁₀-LOV543m3-WH1) and 8 µM (H₁₀-LOV543m3-WH1-mCherry). Each sample was distributed between two centrifuge cells: one of them was scanned at 275 nm (for the double chimera) or 590 nm (for the triple chimera) as the dark state, and the other was illuminated at 450 nm as the lit state. Sedimentation coefficients distributions were calculated with SEDFIT.

Circular dichroism spectroscopy was performed with the chimeras in a Jasco 720 spectropolarimeter, with 150 µL (2.5 µM) of the protein samples in SEC buffer. Proteins were set in 0.1 cm path length quartz cuvettes hold at 20 ºC, and 7 spectra were acquired, in the darkness, at 50 nm·min⁻¹ and accumulated for signal averaging. Protein stability was estimated by thermal denaturation, measuring the variation of ellipticity (θ) at 220 nm with the increase of temperature (20-90 ºC).

## Claims

1. A fusion polypeptide comprising the mutated amino acid sequence of the LOV2 domain shown in SEQ ID NO: 4 fused by its C-terminal end to the N-terminal end of the RepA-WH1 protein shown in SEQ ID NO: 5, wherein said fusion polypeptide comprises the amino acid sequence of SEQ ID NO: 1.

2. The fusion polypeptide according to claim 1, wherein the amino acid sequence of SEQ ID NO: 1 is fused, in its C-terminal end, to the amino acid sequence of the mCherry fluorescent protein probe.

3. The fusion polypeptide according to claim 2, wherein the amino acid sequence of the mCherry fluorescent protein probe is SEQ ID NO: 2.

4. The fusion polypeptide according to claim 3, which comprises the amino acid sequence SEQ ID NO: 3.

5. The fusion polypeptide according to claim 4, which consists of the amino acid sequence SEQ ID NO: 3.

6. A nucleic acid sequence encoding the fusion polypeptide according to any one of claims 1 to 5.

7. A genetic construct comprising the nucleic acid sequence according to claim 6, preferably wherein the genetic construct is a phage.

8. A cell comprising the fusion polypeptide according to any one of claims 1 to 5, the nucleic acid sequence according to claim 6 or the genetic construct according to claim 7.

9. The cell according to claim 8 which is a prokaryotic cell, preferably a bacterial cell.

10. Use of the cell according to claims 8 or 9 for the production of the fusion polypeptide according to any one of claims 1 to 5.

11. *In vitro* use of the fusion polypeptide according to any one of claims 1 to 5, the nucleic acid sequence according to claim 6 or the genetic construct according to claim 7 for inducing the formation of cytotoxic amyloid oligomers in a cell, preferably in a bacterial cell.

12. *In vitro* use according to claim 11, which comprises exposing the cell to blue light.

13. The fusion polypeptide according to any one of claims 1 to 5, the nucleic acid sequence according to claim 6, the genetic construct according to claim 7 or the cell according to claims 8 or 9 for use as a medicament.

14. The fusion polypeptide according to any one of claims 1 to 5, the nucleic acid sequence according to claim 6, the genetic construct according to claim 7 or the cell according to claims 8 or 9 for use in the treatment or prevention of microbial infections, preferably bacterial infections.

15. An *in vitro* method for inducing the formation of cytotoxic amyloid oligomers in a cell, preferably in a bacterial cell, which comprises:
a. expressing the fusion polypeptide according to any one of claims 1 to 5 in the cell, and
b. exposing the cell of step (a) to blue light.
